# EUROPEAN PATENT APPLICATION

(11) **EP 2 789 229 A1**
(43) Date of publication of application: **15.10.2014**
(21) Application number: 13163214.3
(22) Date of filing: 10.04.2013
(51) Int. Cl.: A01K 67/027, C07K 14/195, C12N 15/62, C12N 15/82, C12N 15/85, C12N 15/873

(54) **RALEN-mediated genetic modification techniques**

(71) Applicant: Helmholtz Zentrum München Deutsches Forschungszentrum für Gesundheit und Umwelt (GmbH), 85764 Neuherberg (DE)
(72) Inventor: Kühn, Ralf, 85354 Freising (DE); Wurst, Wolfgang, 80937 München (DE)
(74) Representative: Vossius & Partner

(57) **Abstract**

The present invention relates to a method of producing a eukaryotic cell carrying a modified target sequence in its genome, the method comprising the step: (a) introducing into the cell a fusion protein comprising a DNA-binding domain of a *Ralstonia solanacearum* TALE-like protein (RTL) effector protein and a non-specific cleavage domain of a restriction nuclease or a nucleic acid molecule encoding the fusion protein in expressible form, wherein the fusion protein specifically binds within the target sequence and introduces a double strand break within the target sequence. The present invention further relates to the method of the invention, wherein the modification of the target sequence is by homologous recombination with a donor nucleic acid sequence further comprising the step: (b) introducing a nucleic acid molecule into the cell, wherein the nucleic acid molecule comprises the donor nucleic acid sequence and regions homologous to the target sequence. The present invention also relates to a method of producing a non-human mammal or vertebrate carrying a modified target sequence in its genome. Furthermore, the present invention relates to a fusion protein comprising a RTL effector protein and a non-specific cleavage domain of a restriction nuclease (RALEN).

## Description

The present invention relates to a method of producing a eukaryotic cell carrying a modified target sequence in its genome, the method comprising the step: (a) introducing into the cell a fusion protein comprising a DNA-binding domain of a *Ralstonia solanacearum* TALE-like protein (RTL) effector protein and a non-specific cleavage domain of a restriction nuclease or a nucleic acid molecule encoding the fusion protein in expressible form, wherein the fusion protein specifically binds within the target sequence and introduces a double strand break within the target sequence. The present invention further relates to the method of the invention, wherein the modification of the target sequence is by homologous recombination with a donor nucleic acid sequence further comprising the step: (b) introducing a nucleic acid molecule into the cell, wherein the nucleic acid molecule comprises the donor nucleic acid sequence and regions homologous to the target sequence. The present invention also relates to a method of producing a non-human mammal or vertebrate carrying a modified target sequence in its genome. Furthermore, the present invention relates to a fusion protein comprising a RTL effector protein and a non-specific cleavage domain of a restriction nuclease (RALEN).

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Gene targeting in embryonic stem (ES) cells is routinely applied to modify the mammalian genome, in particular the mouse genome, which established the mouse as the most commonly used genetic animal model (Capecchi, 2005). The basis for reverse mouse genetics was initially established in the 1980-ies, when ES cell lines were established from cultured murine blastocysts, culture conditions were identified that maintain their pluripotent differentiation state *in vitro* (Evans and Kaufman, 1981) and it was found that ES cells are able to colonize the germ line in chimaeric mice upon microinjection into blastocysts (Bradley et al., 1984) (Gossler et al., 1986). Since the first demonstration of homologous recombination in ES cells in 1987 (Thomas and Capecchi, 1987) and the establishment of the first knockout mouse strain in 1989 (Schwartzberg et al., 1989) gene targeting was adopted to a plurality of genes and has been used in the last decades to generate more than 3000 knockout mouse strains that provided a wealth of information on *in vivo* gene functions (Collins et al., 2007)(Capecchi, 2005). Accordingly, gene targeting in ES cells has revolutionised the *in vivo* analysis of mammalian gene function using the mouse as genetic model system. However, at present this reverse genetics approach is restricted to mice, as germ line competent ES cell lines that can be genetically modified could be established only from these animals, so far. The exception from this rule is achieved by homologous recombination in primary cells from pig and sheep followed by the transplantation of nuclei from recombined somatic cells into enucleated oocytes (cloning) (Lai and Prather, 2003)(Gong and Rong, 2003). However, since this methodology is inefficient and time consuming it did not develop into a simple routine procedure.

Although the generation of targeted mouse mutants as described above is by now well established as a routine procedure, this approach has the drawback that it usually requires a long time of hands on work for vector construction, ES cell culture and selection and the breeding of chimaeras. Additional problems that are often encountered during a gene targeting project are the low efficiency of homologous recombination in ES cells and the loss of the germ line competence of ES cells during the long *in vitro* culture and selection phase. Therefore, the successful generation of even a single line of knockout mice requires considerable time, the combined efforts of specialists in molecular biology, ES cell culture and embryo manipulation, and the associated technical infrastructure.

Experiments in model systems have demonstrated that the frequency of homologous recombination of a gene targeting vector is strongly increased if a double strand break is induced within its chromosomal target sequence (Rouet et al., 1994a)(Rouet et al., 1994b). In the absence of a gene targeting vector for homology directed repair, the cells frequently close the break by non-homologous end-joining (NHEJ). Since this mechanism is error-prone it frequently leads to the deletion or insertion of multiple nucleotides at the cleavage site. If the cleavage site is located within the coding region of a gene it is thereby possible to identify and select mutants that exhibit reading frameshift mutations from a mutagenised population and that represent non-functional knockout alleles of the targeted gene.

Direct genome editing by zinc-finger nucleases (ZFN) as well as TAL-nucleases in one-cell embryos has been recently established as a double strand break-based mutagenesis approach in mice, rats, rabbits and zebrafish (Carbery et al., 2010) (Doyon et al., 2008) (Cui et al., 2011) (Flisikowska et al., 2011) (Meyer et al., 2010) (Geurts et al., 2009) (Tesson et al., 2011). Such nucleases are designed to induce double-strand breaks (DSBs) at preselected genomic target sites (Klug, 2010) (Porteus and Carroll, 2005) (Porteus and Baltimore, 2003) (Santiago et al., 2008). DSBs targeted to coding exons frequently undergo sequence deletions leading to gene knockout or allow the insertion (knock-in) of DNA sequences from gene targeting vectors via homologous recombination (HR). The generation of knockout and knock-in mutants at the Rosa26, Mdr1 a, Pxr, and IgM loci by microinjection of ZFNs one-cell embryos of mice, rats and rabbits (Cui et al., 2011) (Flisikowska et al., 2011) (Meyer et al., 2010) (Tesson et al., 2011) has recently been reported.

However, even though the use of zinc finger nucleases results in a higher frequency of homologous recombination, considerable efforts and time are required to design zinc finger proteins that bind a new DNA target sequence at high efficiency. In addition, it has been calculated that using the presently available resources only one zinc finger nuclease could be found within a target region of 1000 basepairs of the mammalian genome (Maeder et al., 2009) (Maeder et al., 2008).

WO2011/05139 describes a method for modifying a target sequence in the genome of a mammalian or avian oocyte by homologous recombination using a zinc finger nuclease and, thus, a method of producing a non-human mammal carrying a modified target sequence in its genome. However, since this method makes use of a zinc finger protein, it is associated with the drawbacks described above with regard to zinc finger proteins. No indication is provided in WO2011/051390 that successful recombination in oocytes, or other eukaryotic cells, could be achieved by any other means but zinc finger proteins.

In addition, TAL elements have been combined with the Fokl nuclease domain to create TAL-nuclease fusion proteins (TALENs) that enable to generate double-strand breaks within intended target regions (Christian et al., 2010) (Miller et al., 2011). TALENs were shown to enable gene editing in mammalian cell lines and in zebrafish, mouse and rat embryos (Sung et al., 2013) (Tesson et al., 2011) (Reyon et al., 2012).

So far, the principles of the TAL peptide DNA recognition only permit a limited collection of di-residues to recognize DNA nucleotides. In addition, TALENs often exhibit a low affinity to the predicted target sequence, thus pointing to an insufficient functionality of the Tal effector di-residue code and often leading to the necessity of time- and cost-consuming further experimentations in order to optimize the respective TALENs.

WO2011/154393 describes a method of modifying a target sequence in the genome of a eukaryotic cell, wherein a fusion protein comprising a DNA-binding domain of a Tal effector protein and a non-specific cleavage domain of a restriction nuclease is employed to introduce a double strand break within the target sequence, thereby enhancing the modification of the target sequence by homologous recombination. It is further described that the method can be applied to oocytes and that it can be used to produce a non-human mammal or vertebrate carrying a modified target sequence in its genome. However, the only methods for introducing double strand breaks and enhancing the frequency of homologous recombination that are described in WO2011/154393 are the use of zinc finger proteins or fusion proteins comprising a DNA-binding domain of a Tal effector proteins and a non-specific cleavage domain of a restriction nuclease. No indication is provided that successful recombination in eukaryotic cells could be achieved by any other means but zinc finger or Tal effector proteins and, in particular, no reference is made to the *Ralstonia solanacarum* derived RTL repeat proteins and their unique DNA recognition code.

Recently, a novel system for DNA recognition was described from *Ralstonia solanacearum* type III effector proteins that employ a new type of RTL protein repeats and a new di-residue code for DNA recognition (Li et al., 2013). However, this system has only been shown to be functional for transcriptional activation in plant cells such as tobacco cells, but not for vertebrate cells, mammalian cells, or oocytes. Moreover, due to large taxonomical differences, it is entirely unclear whether this system may be used in fusion with nuclease domains.

The technical problem underlying the present invention is thus the provision of improved means and methods for modifying the genome of eukaryotic cells, such as e.g. mammalian or vertebrate cells.

The solution to this technical problem is achieved by providing the embodiments characterised in the claims.

Accordingly, the present invention relates to a method of producing a eukaryotic cell carrying a modified target sequence in its genome, the method comprising the step: (a) introducing into the cell a fusion protein comprising a DNA-binding domain of a *Ralstonia solanacearum* TALE-like protein (RTL) effector protein and a non-specific cleavage domain of a restriction nuclease or a nucleic acid molecule encoding the fusion protein in expressible form, wherein the fusion protein specifically binds within the target sequence and introduces a double strand break within the target sequence.

The term "eukaryotic cell" as used herein, refers to any cell of a unicellular or multi-cellular eukaryotic organism, including cells from animals like vertebrates and mammals as well as from fungi.

In accordance with the present invention, a "modified target sequence" is a nucleotide sequence in which genomic manipulations have led to an alteration of the respective target nucleotide sequence. The term "target sequence in the genome", as used herein, refers to the genomic location that is to be modified by the method of the invention. The "target sequence in the genome" comprises but is not restricted to the nucleotide(s) subject to the particular modification, i.e. the "target sequence in the genome" also comprises the sequence surrounding the relevant nucleotide(s) to be modified. Preferably the "target sequence in the genome" also comprises at least 10, such as at least 100, such as at least 200, such as at least 500, such as at least 1000 nucleotide(s) upstream and/or downstream of the relevant nucleotide(s) to be modified.

More preferably, the term "target sequence" refers to the entire gene to be modified.

The term "modified" includes, but is not limited to, one or more nucleotides that are substituted, inserted and deleted within the target sequence.

The term "substitution", as used herein, is defined in accordance with the pertinent art and refers to the replacement of nucleotides with other nucleotides. The term includes for example the replacement of single nucleotides resulting in point mutations. Said point mutations can lead to an amino acid exchange in the resulting protein product but may also not be reflected on the amino acid level (i.e. silent mutations). Also encompassed by the term "substitution" are mutations resulting in the replacement of multiple nucleotides, such as for example parts of genes, such as parts of exons or introns as well as the replacement of entire genes. The number of nucleotides that replace the originally present nucleotides may be the same or different (i.e. more or less) as compared to the number of nucleotides removed. Preferably, the number of replacement nucleotides corresponds to the number of originally present nucleotides that are substituted.

The term "insertion", in accordance with the present invention, is defined in accordance with the pertinent art and refers to the incorporation of one or more nucleotides into a nucleic acid molecule. Insertion of parts of genes, such as parts of exons or introns as well as insertion of entire genes is also encompassed by the term "insertion". When the number of inserted nucleotides is not dividable by three, the insertion can result in a frameshift mutation within a coding sequence of a gene. Such frameshift mutations will alter the amino acids encoded by a gene following the mutation. In some cases, such a mutation will cause the active translation of the gene to encounter a premature stop codon, resulting in an end to translation and the production of a truncated protein. When the number of inserted nucleotides is instead dividable by three, the resulting insertion is an "in-frame insertion". In this case, the reading frame remains intact after the insertion and translation will most likely run to completion if the inserted nucleotides do not code for a stop codon. However, because of the inserted nucleotides, the finished protein will contain, depending on the size of the insertion, one or multiple new amino acids that may affect the function of the protein.

The term "deletion", as used in accordance with the present invention, is defined in accordance with the pertinent art and refers to the loss of nucleotides or larger parts of genes, such as exons or introns as well as entire genes. As defined with regard to the term "insertion", the deletion of a number of nucleotides that is not evenly dividable by three will lead to a frameshift mutation, causing all of the codons occurring after the deletion to be read incorrectly during translation, potentially producing a severely altered and most likely non-functional protein. If a deletion does not result in a frameshift mutation, i.e. because the number of nucleotides deleted is dividable by three, the resulting protein is nonetheless altered as the finished protein will lack, depending on the size of the deletion, one or several amino acids that may affect the function of the protein.

The above defined modifications are not restricted to coding regions in the genome, but can also be introduced into non-coding regions of the target genome, for example in regulatory regions such as promoter or enhancer elements or in introns.

Examples of modifications of the target genome include both targeted and random modifications, such as e.g. the introduction of mutations into a wildtype gene in order to analyse its effect on gene function; the replacement of an entire gene with a mutated gene or, alternatively, if the target sequence comprises mutation(s), the alteration of these mutations to identify which one is causative of a particular effect; the removal of entire genes or proteins or the removal of regulatory elements from genes or proteins as well as the introduction of fusion-partners, such as for example purification tags such as the his-tag or the tap-tag.

The term "introducing into the cell", as used herein, relates to any known method of bringing a protein or a nucleic acid molecule into a cell. Non-limiting examples include microinjection, infection with viral vectors, electroporation and the formulation with cationic lipids. All these methods are well known in the art.

The term "fusion protein comprising a DNA-binding domain of a *Ralstonia solanacearum* TALE-like protein (RTL) effector protein and a non-specific cleavage domain of a restriction nuclease", as used in accordance with the present invention and as also referred to herein as RALEN, refers to a fusion protein comprising a DNA-binding domain, wherein the DNA-binding domain comprises or consists of RTL effector motifs, and the non-specific cleavage domain of a restriction nuclease. The fusion protein employed in the method of the invention retains or essentially retains the enzymatic activity of the native (restriction) endonuclease. In accordance with the present invention, (restriction) endonuclease function is essentially retained if at least 60% of the biological activity of the endonuclease activity is retained. Preferably, at least 75%, such as at least 80% of the endonuclease activity are retained. More preferred is that at least 90%, such as at least 95%, even more preferred at least 98%, such as at least 99% of the biological activity of the endonuclease is retained. Most preferred is that the biological activity is fully, i.e. to 100%, retained. Also in accordance with the invention are fusion proteins having an increased biological activity compared to the endogenous endonuclease, i.e. having more than 100% activity. Methods of assessing biological activity of (restriction) endonucleases are well known to the person skilled in the art and include, without being limiting, the incubation of an endonuclease with recombinant DNA and the analysis of the reaction products by gel electrophoresis (Bloch, 2001).

The term "RTL effector protein", as used herein, refers to proteins belonging to the type III effector family of proteins expressed by bacterial plant pathogens of the genus *Ralstonia* (Genin and Denny, 2012)(Salanoubat et al., 2002). The pathogenicity of many bacteria depends on the injection of effector proteins via type III secretion into eukaryotic cells in order to manipulate cellular processes. Members of the large type III effector family are virulence factors of *Ralstonia* (Remigi et al., 2011) and some members contain protein domains contain repeat sequence motifs (Heuer et al., 2007) that act as transcriptional activators that likely manipulate the defense response of their host by mimicking eukaryotic transcription factors (Li et al., 2013). RTL effector proteins are characterized by a central domain of tandem repeats, i.e. a DNA-binding domain as well as nuclear localization signals (NLSs) and an acidic transcriptional activation domain. Members of this effector family are related and differ in the amino acid sequence of their repeats and in the number of repeats. The number and order of repeats in a RTL effector protein likely determine its specific activity. These repeats are referred to herein as "RTL repeats". One exemplary member of this effector family, YP_003750492 (SEQ ID NO:5) from the *Ralstonia solanacearum* strain PSI07, contains 13 repeats that are essential for DNA binding and represent a distinct type of DNA binding domain.
The mechanism of sequence specific DNA recognition has been elucidated by a recent study on RTL repeats that revealed a part of the RTL effectors' DNA recognition code (Li et al., 2013). RTL effector motifs or repeats are 35 amino acid protein sequence motifs. The amino acid sequences of the repeats are conserved, except for two adjacent, highly variable residues (at positions 12 and 13) that determine specificity towards the DNA base A, G, C or T or combinations thereof. In other words, binding to DNA is mediated by contacting a nucleotide of the DNA double helix with the variable residues at position 12 and 13 within the RTL effector repeat. Each RTL effector repeat primarily recognizes a single nucleotide within the DNA substrate.

In a preferred embodiment, the RTL effector repeat has the sequence of any one of SEQ ID NO:2, 3 or 4 or a sequence wherein the di-residues at position 12 and 13 of SEQ ID NO:2, 3 or 4 is replaced by a di-residue selected from the group consisting of: FF, FL, FS, FY, FC, FW, FP, FH, FQ, FR, FI, FM, FT, FN, FK, FV, FA, FD, FE, FG, LF, LL, LS, LY, LC, LW, LP, LH, LQ, LR, LI, LM, LT, LN, LK, LV, LA, LD, LE, LG, SF, SL, SS, SY, SC, SW, SP, SH, SQ, SR, SI, SM, ST, SN, SK, SV, SA, SD, SE, SG, YF, YL, YS, YY, YC, YW, YP, YH, YQ, YR, YI, YM, YT, YN, YK, YV, YA, YD, YE ,YG, CF, CL, CS, CY, CC, CW, CP, CH, CQ, CR, CI, CM, CT, CN, CK, CV, CA, CD, CE, CG, WF, WL, WS, WY, WC, WW, WP, WH, WQ, WR, WI, WM, WT, WN, WK, WV, WA, WD, WE, WG, PF, PL, PS, PY, PC, PW, PP, PH, PQ, PR, PI, PM, PT, PN, PK, PV, PA, PD, PE, PG, HF, HL, HS, HY, HC, HW, HP, HH, HQ, HR, HI, HM, HT, HK, HV, HA, HE, QF, QL, QS, QY, QC, QW, QP, QH, QQ, QR, QI, QM, QT, QN, QK, QV, QA, QD, QE, QG, RF, RL, RS, RY, RC, RW, RP, RH, RQ, RR, RI, RM, RT, RN, RK, RV, RA, RD, RE, RG, IF, IL, IS, IY, IC, IW, IP, IH, IQ, IR, II, IM, IT, IN, IK, IV, IA, ID, IE, MF, ML, MS, MY, MC, MW, MP, MH, MQ, MR, MI, MM, MT, MN, MK, MV, MA, MD, ME, MG, TF, TL, TS, TY, TC, TW, TP, TH, TQ, TR, TI, TM, TT, TN, TK, TV, TA, TD, TE, TG, NF, NL, NY, NC, NW, NP, NH, NQ, NR, NM, NV, NA, NE, KF, KL, KS, KY, KC, KW, KP, KH, KQ, KR, KI, KM, KT, KN, KK, KV, KA, KD, KE, KG, VF, VL, VS, VY, VC, VW, VP, VH, VQ, VR, VI, VM, VT, VN, VK, VV, VA, VD, VE, VG, AF, AL, AS, AY, AC, AW, AP, AH, AQ, AR, AI, AM, AT, AN, AK, AV, AA, AD, AE, AG, DF, DL, DS, DY, DC, DW, DP, DH, DQ, DR, DI, DM, DT, DN, DK, DV, DA, DD, DE, DG, EF, EL, ES, EY, EC, EW, EP, EH, EQ, ER, EI, EM, ET, EN, EK, EV, EA, ED, EE, EG, GF, GL, GS, GY, GC, GW, GP, GH, GQ, GR, GI, GM, GT, GN, GK, GV, GA, GD, GE, GG, HD, NI, NG, NS, NN, IG, HG and NK (see SEQ ID NO:32).
More preferably, the di-residue at position 12 and 13 of SEQ ID NO:2, 3 or 4 is replaced by a di-residue selected from the group consisting of: FF, FL, FS, FY, FC, FW, FP, FH, FQ, FR, FI, FM, FT, FN, FK, FV, FA, FD, FE, FG, LF, LL, LS, LY, LC, LW, LP, LH, LQ, LR, LI, LM, LT, LN, LK, LV, LA, LD, LE, LG, SF, SL, SS, SY, SC, SW, SP, SH, SQ, SR, SI, SM, ST, SN, SK, SV, SA, SD, SE, SG, YF, YL, YS, YY, YC, YW, YP, YH, YQ, YR, YI, YM, YT, YN, YK, YV, YA, YD, YE ,YG, CF, CL, CS, CY, CC, CW, CP, CH, CQ, CR, CI, CM, CT, CN, CK, CV, CA, CD, CE, CG, WF, WL, WS, WY, WC, WW, WP, WH, WQ, WR, WI, WM, WT, WN, WK, WV, WA, WD, WE, WG, PF, PL, PS, PY, PC, PW, PP, PH, PQ, PR, PI, PM, PT, PN, PK, PV, PA, PD, PE, PG, HF, HL, HS, HY, HC, HW, HP, HH, HQ, HR, HI, HM, HT, HK, HV, HA, HE, QF, QL, QS, QY, QC, QW, QP, QH, QQ, QR, QI, QM, QT, QN, QK, QV, QA, QD, QE, QG, RF, RL, RS, RY, RC, RW, RP, RH, RQ, RR, RI, RM, RT, RN, RK, RV, RA, RD, RE, RG, IF, IL, IS, IY, IC, IW, IP, IH, IQ, IR, II, IM, IT, IN, IK, IV, IA, ID, IE, MF, ML, MS, MY, MC, MW, MP, MH, MQ, MR, MI, MM, MT, MN, MK, MV, MA, MD, ME, MG, TF, TL, TS, TY, TC, TW, TP, TH, TQ, TR, TI, TM, TT, TN, TK, TV, TA, TD, TE, TG, NF, NL, NY, NC, NW, NP, NH, NQ, NR, NM, NV, NA, NE, KF, KL, KS, KY, KC, KW, KP, KH, KQ, KR, KI, KM, KT, KN, KK, KV, KA, KD, KE, KG, VF, VL, VS, VY, VC, VW, VP, VH, VQ, VR, VI, VM, VT, VN, VK, VV, VA, VD, VE, VG, AF, AL, AS, AY, AC, AW, AP, AH, AQ, AR, AI, AM, AT, AN, AK, AV, AA, AD, AE, AG, DF, DL, DS, DY, DC, DW, DP, DH, DQ, DR, DI, DM, DT, DN, DK, DV, DA, DD, DE, DG, EF, EL, ES, EY, EC, EW, EP, EH, EQ, ER, EI, EM, ET, EN, EK, EV, EA, ED, EE, EG, GF, GL, GS, GY, GC, GW, GP, GH, GQ, GR, GI, GM, GT, GN, GK, GV, GA, GD, GE and GG.

Notably, the RTL repeat DNA recognition code is distinguished from the restricted DNA binding code found in *Xanthomonas*-derived TAL effector proteins (Boch et al., 2009). For example, it has been found that the RTL repeat di-residue of asparagine and aspartic acid (i.e. ND) mediate the recognition of cytidine and that the di-residue of histidine and asparagine (i.e. HN) mediate the recognition of adenine and guanine. In contrast, it has been found that *Xanthomonas* Tal repeats use the di-residue of histidine and aspartic acid (i.e. HD) to recognize cytidine and the di-residue of asparagines and asparagines (i.e. NN) to recognize adenine and guanine (Boch et al., 2009).
Accordingly, it is preferred herein that the di-residue HN is employed in RTL effector repeats in order to ensure binding to adenine or guanine, ND is employed in RTL repeats in order to ensure binding to cytidine and NT is employed in RTL repeats in order to ensure binding to thymidine.

Binding to longer DNA sequences is achieved by linking several of these RTL effector repeats, each distinguished by a di-residue at position 12/13 that specifies binding to a selected target nucleotide, in tandem to form a "DNA-binding domain of a RTL effector protein". Two of such DNA binding domains, each fused to a Fokl nuclease domain, are preferably used together to create a double-strand break at the selected target region of the genome. Accordingly, the term "introducing into the cell a fusion protein", as used herein, is not restricted to the use of exactly one molecular type of such a fusion protein, but also encompasses the use of more than one, such as for example two different molecular types of such fusion proteins comprising a DNA-binding domain of a *Ralstonia solanacearum* TALE-like (RTL) effector protein and a non-specific cleavage domain of a restriction nuclease. Also encompassed are the use of three, four, five, six, seven, eight etc. different molecular types of of such fusion proteins. Preferably, two different fusion proteins are employed, as shown in the appended examples.
The term "DNA-binding domain of a RTL effector protein" relates to both DNA-binding domains found in naturally occurring RTL effector proteins as well as to DNA-binding domains designed to bind to a specific target nucleotide sequence, such as e.g. described in the examples below. Creation of DNA-binding domains of RTL repeats that can specifically recognize a particular target enables the use of said DNA-binding domains of RTL effector proteins for the creation of RTL repeat - nuclease fusion proteins (RALEN) that recognize and cleave a specific target sequence. Preferably, the DNA-binding domain is derived from the RTL effector repeats found in naturally occurring RTL effector proteins, wherein the amino acids at position 12 and 13 are selected (and, where necessary, replaced) in order to providing binding specificity to a specific nucleotide. Such naturally occurring RTL effector proteins include, for example, repeats of RTL effector proteins selected from the group consisting of YP_003750492 (SEQ ID NO:5), RSc1815 (SEQ ID NO:25), CAQ18687.1 (SEQ ID NO:26), CCA82456.1 (SEQ ID NO:27), ABO27067.1 (SEQ ID NO:28), ABO27073.1 (SEQ ID NO:29), ABO27070.1 (SEQ ID NO:30), ABO27069.1 (SEQ ID NO:31), ABO27071.1 (SEQ ID NO:10), ABO27074.1 (SEQ ID NO:12), ABO27068.1 (SEQ ID NO:13), ABO27072.1 (SEQ ID NO:14) and ABO27075.1 (SEQ ID NO:15).

Preferably, the restriction nuclease is an endonuclease. The terms "endonuclease" and "restriction endonuclease" are used herein according to the well-known definitions provided by the art. Both terms thus refer to enzymes capable of cutting nucleic acids by cleaving the phosphodiester bond within a polynucleotide chain. Preferably, the endonuclease is a type II S restriction endonuclease, such as for example FokI, AlwI, SfaNI, SapI, PleI, NmeAIII, MboII, MlyI, MmeI, HpYAV, HphI, HgaI, FauI, EarI, EciI, BtgZI, CspCI, BspQI, BspMI, BsaXI, BsgI, BseI, BpuEIBmrIBcgIBbvI, BaeI, BbsIAlwI, or AcuI or a type III restriction endonuclease (e.g. EcoP1I, EcoP15I, HinfIII). Also envisaged herein are meganucleases, such as for example I-Scel.
More preferably, the endonuclease is FokI endonuclease. Fokl is a bacterial type IIS restriction endonuclease. It recognises the non-palindromic penta-deoxyribonucleotide 5'-GGATG-3': 5'-CATCC-3' in duplex DNA and cleaves 9/13 nucleotides downstream of the recognition site. Fokl does not recognise any specific-sequence at the site of cleavage. Once the DNA-binding domain (either of the naturally occurring endonuclease, e.g. FokI or, in accordance with the present invention, of the fusion protein comprising a DNA-binding domain of a RTL effector protein and a nuclease domain) is anchored at the recognition site, a signal is transmitted to the endonuclease domain and cleavage occurs. The distance of the cleavage site to the DNA-binding site of the fusion protein depends on the particular endonuclease present in the fusion protein. For example, the fusion protein employed in the examples of the present invention cleaves in the middle of a 6 bp sequence that is flanked by the two binding sites of the fusion protein. As a further example, naturally occurring endonucleases such as Fokl and EcoP15I cut at 9/13 and 27 bp distance from the DNA binding site, respectively.

In accordance with the present invention, fusion proteins are envisaged that are provided as functional monomers comprising a DNA-binding domain of a RTL effector protein coupled with a single nuclease domain. The DNA-binding domain of a RTL effector protein and the cleavage domain of the nuclease may be directly fused to one another or may be fused via a linker.

The term "linker', as used in accordance with the present invention, relates to a sequel of amino acids (i.e. peptide linkers) as well as to non-peptide linkers.

Peptide linkers as envisaged by the present invention are (poly)peptide linkers of at least 1 amino acid in length. Preferably, the linkers are 1 to 100 amino acids in length. More preferably, the linkers are 5 to 50 amino acids in length and even more preferably, the linkers are 10 to 20 amino acids in length. It is well known to the skilled person that the nature, i.e. the length and/or amino acid sequence of the linker may modify or enhance the stability and/or solubility of the molecule. Thus, the length and sequence of a linker depends on the composition of the respective portions of the fusion protein of the invention. The skilled person is aware of methods to test the suitability of different linkers. For example, the properties of the molecule can easily be tested by testing the nuclease activity as well as the DNA-binding specificity of the respective portions of the fusion protein of the invention.

It will be appreciated by the skilled person that when the fusion protein of the invention is provided as a nucleic acid molecule encoding the fusion protein in expressible form, the linker is a peptide linker also encoded by said nucleic acid molecule.

The term "non-peptide linker", as used in accordance with the present invention, refers to linkage groups having two or more reactive groups but excluding peptide linkers as defined above. For example, the non-peptide linker may be a polymer having reactive groups at both ends, which individually bind to reactive groups of the individual portions of the fusion protein of the invention, for example, an amino terminus, a lysine residue, a histidine residue or a cysteine residue. The reactive groups of the polymer include an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide polymer may be the same or different. For example, the non-peptide polymer may have a maleimide group at one end and an aldehyde group at another end.

In a preferred embodiment, the linker is a peptide linker.

Without wishing to be bound by theory, the present inventors believe that the mechanism of double-strand cleavage by a fusion protein of the invention requires dimerisation of the nuclease domain in order to cut the DNA substrate. Thus, in a preferred embodiment, at least two fusion proteins are introduced into the cell in step (a). Dimerisation of the fusion protein can result in the formation of homodimers if only one type of fusion protein is present or in the formation of heterodimers, when different types of fusion proteins are present. It is preferred in accordance with the present invention that at least two different types of fusion proteins having differing DNA-binding domains of a RTL effector protein are introduced into the cell. The at least two different types of fusion proteins can be introduced into the cell either separately or together. Also envisaged herein is a fusion protein, which is provided as a functional dimer via linkage of two subunits of identical or different fusion proteins prior to introduction into the cell. Suitable linkers have been defined above.

The term "nucleic acid molecule encoding the fusion protein in expressible form" refers to a nucleic acid molecule which, upon expression in a cell or a cell-free system, results in a functional fusion protein. Means and methods to ensure expression of a functional fusion protein are well known in the art. For example, the coding sequences may be comprised in a vector, such as for example a plasmid, cosmid, virus, bacteriophage or another vector used conventionally e.g. in genetic engineering. The coding sequences inserted in the vector can e.g. be synthesized by standard methods, or isolated from natural sources. The coding sequences may further be ligated to transcriptional regulatory elements and/or to other amino acid encoding sequences. Such regulatory sequences are well known to those skilled in the art and include, without being limiting, regulatory sequences ensuring the initiation of transcription, internal ribosomal entry sites (IRES) (Owens, Proc. Natl. Acad. Sci. USA 98 (2001), 1471-1476) and optionally regulatory elements ensuring termination of transcription and stabilization of the transcript. Non-limiting examples for regulatory elements ensuring the initiation of transcription comprise a translation initiation codon, transcriptional enhancers such as e.g. the SV40-enhancer, insulators and/or promoters, such as for example the cytomegalovirus (CMV) promoter, SV40-promoter, RSV-promoter (Rous sarcome virus), the IacZ promoter, chicken beta-actin promoter, CAG-promoter (a combination of chicken beta-actin promoter and cytomegalovirus immediate-early enhancer), the gai10 promoter, human elongation factor 1α-promoter, AOX1 promoter, GAL1 promoter CaM-kinase promoter, the lac, trp or tac promoter, the IacUV5 promoter, the autographa californica multiple nuclear polyhedrosis virus (AcMNPV) polyhedral promoter or a globin intron in mammalian and other animal cells. Non-limiting examples for regulatory elements ensuring transcription termination include the V40-poly-A site, the tk-poly-A site or the SV40, IacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing. Moreover, elements such as origin of replication, drug resistance gene or regulators (as part of an inducible promoter) may also be included. Moreover, the coding sequences may further be ligated to one or more selected from e.g. tags or signal sequences. Examples include a signal sequence for intra- or extracellular targeting, e.g. nuclear localisation signals (NLS; e.g. PPKKKRKV), as well as His-tags, Strep-tags, GST-tags, tandem affinity purification (TAP) tags, biotinylation using the bir BAD system (biotinylation acceptor domain) and HA tags. It is particular preferred in accordance with the present invention that the nucleic acid molecule encoding the fusion protein in expressible form includes a nuclear localisation signal.

Nucleic acid molecules as well as nucleic acid sequences, as used throughout the present description, include DNA, such as cDNA or genomic DNA, and RNA. Preferably, embodiments reciting "RNA" are directed to mRNA. Furthermore included is genomic RNA, such as in case of RNA of RNA viruses.

Also, the nucleic acid molecules used in accordance with the invention may be nucleic acid mimicking molecules known in the art such as synthetic or semi-synthetic derivatives of nucleic acid molecules and mixed polymers. They may contain additional non-natural or derivatised nucleotide bases, as will be readily appreciated by those skilled in the art. Nucleic acid mimicking molecules or nucleic acid derivatives according to the invention include, without being limiting, phosphorothioate nucleic acid, phosphoramidate nucleic acid, morpholino nucleic acid, hexitol nucleic acid (HNA), peptide nucleic acid (PNA) and locked nucleic acid (LNA).

It will be readily appreciated by the skilled person that more than one nucleic acid molecule may encode a fusion protein in accordance with the present invention due to the degeneracy of the genetic code. Degeneracy results because a triplet code designates 20 amino acids and a stop codon. Because four bases exist which are utilized to encode genetic information, triplet codons are required to produce at least 21 different codes. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having different sequences, but still encoding the same fusion protein can be employed in accordance with the present invention.

The nucleic acid molecules used in accordance with the present invention may be of natural as well as of (semi) synthetic origin. Thus, the nucleic acid molecules may, for example, be nucleic acid molecules that have been synthesised according to conventional protocols of organic chemistry. The person skilled in the art is familiar with the preparation and the use of said probes (see, e.g., Sambrook and Russel "Molecular Cloning, A Laboratory Manual", Cold Spring Harbor Laboratory, N.Y. (2001)).

In accordance with the present invention, the term "specifically binds within the target sequence and introduces a double strand break within the target sequence" means that the fusion protein is designed such that statistically it only binds to a particular sequence (i.e. the target sequence) and does not bind to an unrelated sequence elsewhere in the genome. Preferably, the fusion protein in accordance with the present invention comprises at least 18 RTL effector motifs. In other words, the DNA-binding domain of a RTL effector protein within said fusion protein is comprised of at least 18 RTL effector motifs. In the case of fusion proteins consisting of dimers, as described above, this means that each fusion protein monomer comprises at least nine RTL effector motifs. More preferably, each fusion protein comprises at least 12 RTL effector motifs, such as for example at least 14 or at least 16 RTL effector motifs. Methods for testing the DNA-binding specificity of a fusion protein in accordance with the present invention are known to the skilled person and include, without being limiting, transcriptional reporter gene assays and electrophoretic mobility shift assays (EMSA).

The term "introduces a double strand break within the target sequence" relates to the interruption of the DNA strands of a DNA double helix, wherein the two strands in the double helix are severed.

The presence of such a double strand break within the genomic DNA triggers intracellular repair mechanisms. Typically, such double strand breaks are repaired by either nonhomologous end joining (NHEJ) or homologous recombination.

Preferably, the binding site of the fusion protein is up to 500 nucleotides, such as up to 250 nucleotides, up to 100 nucleotides, up to 50 nucleotides, up to 25 nucleotides, up to 10 nucleotides such as up to 5 nucleotides upstream (i.e. 5') or downstream (i.e. 3') of the nucleotide(s) that is/are modified in accordance with the present invention.

In accordance with the present invention, a novel method is provided for introducing gene modifications, including targeted gene modifications, into the genome of eukaryotic cells. Said method provides a high frequency of successful genetic modification by employing a fusion protein comprising a DNA-binding domain of a RTL effector repeats and a non-specific cleavage domain of a restriction nuclease. Performing the cleavage step of the method of the invention will frequently lead to spontaneous genome modifications through nucleotide loss associated with the repair of double strand breaks by nonhomologous end joining (NHEJ) repair. In addition, by providing a nucleic acid molecule comprising a donor nucleic acid sequence and regions homologous to the target sequence, targeted modification of a genome can be achieved with high specificity.

Several methods have been described in the art for achieving an improved frequency of genetic modification. Such methods include, for example, the use of zinc finger nucleases for achieving homologous recombination. However, in order to design zinc finger proteins that bind a new DNA target sequence at high efficiency, considerable efforts and time are required. Furthermore, neighbouring zinc fingers generally influence each other. Thus, they cannot be simply combined into a larger protein in a combinatorial way in order to enhance sequence specificity. As a consequence, the addition of new zinc fingers to a preselected zinc finger protein requires a laborious screening and selection procedure for each individual step. Furthermore, due to the incompletely known DNA binding code and the limited resources of coding zinc finger domains, it is presently difficult to design a nuclease fused to a zinc finger protein specific to any given DNA target sequence. It has been calculated that using the presently available resources only one zinc finger nuclease could be found within a target region of 1000 base-pairs of the mammalian genome (Maeder et al., 2009) (Maeder et al., 2008).

Another method employed to achieve a target sequence specific DNA double strand break is the use of yeast derived meganucleases, representing restriction enzymes like I-Scel that binds to specific 18 bp recognition sequence that does not occur naturally in mammalian genomes. However, a combinatorial code for the DNA binding specificity of meganucleases has not yet been revealed. The redesign of the DNA binding domain of meganucleases allowed so far only the substitution of one or a few nucleotides within their natural binding sequence (Pâques and Duchateau, 2007). Therefore, the choice of meganuclease target sites is very limited and it is presently not possible to design new meganucleases that bind to any preferred target region within mammalian genomes.

In contrast to these methods, the RTL effector DNA binding domains provide a simple combinatorial code for the construction of new DNA binding proteins with chosen specificity that can be applied to any target sequence within any genome. Thus, in accordance with the present invention, a method of introducing genetic modifications into a target genome is provided that overcomes the above discussed problems currently faced by the skilled person. In particular, any number of nucleotide-specific RTL effector repeats can be combined to form a sequence-specific DNA-binding domain to be employed in the fusion protein in accordance with the present invention. Thus, any sequence of interest can now be targeted in a cost-effective, easy and fast way.

Compared to TAL effector proteins described in the art, RTL effector proteins differ significantly in the amino acids of their repeats, i.e. one third of the amino acid of the repeat sequence are different, thus leading the skilled person to the conclusion that the structure and physical constitution and biochemical properties of RTL repeat proteins must be quite distinct. From these differences the skilled person would assume that nuclease fusion proteins employing RTL effector DNA binding domains would be unstable at 37°C within the cytoplasmic milieu of mammalian cells due to negative effects of salt concentrations and the pH value. Further, the skilled person would assume that nuclease fusion proteins employing RTL effector DNA binding domains would be recognized as "foreign" and degraded by mammalian proteases or that they may simply stick to and being trapped by other proteins present in mammalian cells. In addition, RTL effector proteins employ a di-residue code that is distinct from the code employed by TAL effector proteins. Accordingly, the skilled person would expect that the RTL di-residue code, which was described for plant cells, may not be functional in mammalian cells because these "new" di-residues may make contacts to DNA that are potentially weaker than the known TAL di-residues, which negatively affects functionality at the higher temperature of 37°C, or they may be unable to bind the target DNA because the mammalian DNA is unaccesible to them. This is because mammalian genomic DNA is densely packed with mammalian histones, which are highly basic proteins that are further modified by methylation, acidic and basic residues and that may interact with RTL specific residues in an unfavorable manner. For example, the RTL consensus repeats show in comparison to TAL consensus repeats a basic lysine instead of the negative glutamine at position 23, negative glutamine instead of the neutral leucine at position 25, basic arginine instead of polar cysteine at position 30, and polar tyrosine instead of the negative loaded glutamine at position 34.

In a preferred embodiment of the present invention, the modification of the target sequence is by homologous recombination with a donor nucleic acid sequence further comprising the step: (b) introducing a nucleic acid molecule into the cell, wherein the nucleic acid molecule comprises the donor nucleic acid sequence and regions homologous to the target sequence.

The term "homologous recombination", is used according to the definitions provided in the art. Thus, it refers to a mechanism of genetic recombination in which two DNA strands comprising similar nucleotide sequences exchange genetic material. Cells use homologous recombination during meiosis, where it serves to rearrange DNA to create an entirely unique set of haploid chromosomes, but also for the repair of damaged DNA, in particular for the repair of double strand breaks. The mechanism of homologous recombination is well known to the skilled person and has been described, for example by Paques and Haber ( Paques F, Haber JE.; Microbiol Mol Biol Rev 1999; 63:349-404)

In accordance with the present invention, the term "donor nucleic acid sequence" refers to a nucleic acid sequence that serves as a template in the process of homologous recombination and that carries the modification that is to be introduced into the target sequence. By using this donor nucleic acid sequence as a template, the genetic information, including the modifications, is copied into the target sequence within the genome of the cell. In non-limiting examples, the donor nucleic acid sequence can be essentially identical to the part of the target sequence to be replaced, with the exception of one nucleotide which differs and results in the introduction of a point mutation upon homologous recombination or it can consist of an additional gene previously not present in the target sequence. The donor nucleic acid sequence may be a double stranded nucleic acid sequence or a single-stranded nucleic acid molecule.

In accordance with the method of the present invention of producing a eukaryotic cell carrying a modified target sequence in its genome, the nucleic acid molecule introduced into the cell in step (b) comprises the donor nucleic acid sequence as defined above as well as additional regions that are homologous to the target sequence.

The term "regions homologous to the target sequence" (also referred to as "homology arms" herein), in accordance with the present invention, refers to regions having sufficient sequence identity to ensure specific binding to the target sequence. Methods to evaluate the identity level between two nucleic acid sequences are well known in the art. For example, the sequences can be aligned electronically using suitable computer programs known in the art. Such programs comprise BLAST (Altschul et al., 1990), variants thereof such as WU-BLAST (Altschul and Gish, 1996), FASTA (Pearson and Lipman, 1988) or implementations of the Smith-Waterman algorithm (SSEARCH) (Smith and Waterman, 1981). These programs, in addition to providing a pairwise sequence alignment, also report the sequence identity level (usually in percent identity) and the probability for the occurrence of the alignment by chance (P-value). In accordance with the present invention it is preferred that BLAST is used to determine the identify level between two nucleic acid sequences.

Preferably, the "regions homologous to the target sequence" have a sequence identity with the corresponding part of the target sequence of at least 95%, more preferred at least 97%, more preferred at least 98%, more preferred at least 99%, even more preferred at least 99.9% and most preferred 100%. The above defined sequence identities are defined only with respect to those parts of the target sequence which serve as binding sites for the homology arms. Thus, the overall sequence identity between the entire target sequence and the homologous regions of the nucleic acid molecule of step (b) of the method of modifying a target sequence of the present invention can differ from the above defined sequence identities, due to the presence of the part of the target sequence which is to be replaced by the donor nucleic acid sequence. It is preferred that at least two regions homologous to the target sequence are present in the nucleic acid molecule of (b).

In accordance with this preferred embodiment the method of the present invention, step (a) of introducing the fusion protein into the cell and step (b) of introducing the nucleic acid molecule into the cell are either carried out concomitantly, i.e. at the same time or are carried out separately, i.e. individually and at different time points. When the steps are carried out concomitantly, both the fusion protein and the nucleic acid molecule can be administered in parallel, for example using two separate injection needles or can be mixed together and, for example, be injected using one needle.

Accordingly, it will also be appreciated by one of skill in the art that the nucleic acid molecule to be introduced into the cell in steps (a) and (b) may be one nucleic acid molecule comprising both the nucleic acid molecule encoding the fusion protein and the nucleic acid molecule comprising the donor nucleic acid sequence and regions homologous to the target sequence. In that case, steps (a) and (b) would be carried out in one concomitant method step by introducing said one nucleic acid molecule. Alternatively, the nucleic acid molecule of step (b) may be a further nucleic acid molecule, to be introduced in addition to the nucleic acid molecule encoding the fusion protein in accordance with step (a).

In a more preferred embodiment of the method of the invention, the nucleic acid molecule of step (b) is a single stranded oligodesoxynucleotide.

The term "oligodesoxynucleotide", abbreviated as ODN herein, relates to a nucleic acid polymer made up of a sequence of desoxynucleotide residues. An ODN in accordance with the present invention refers to both oligodesoxynucleotides and polydesoxynucleotides and is at least 30 nucleotides in length, such as e.g. at least 40 nucleotides in length, e.g. at least 50 nucleotides in length, such as e.g. at least 60 nucleotides in length, more preferably at least 70 nucleotides in length, such as e.g. at least 80 nucleotides in length, e.g. at least 90 nucleotides in length and even more preferably at least 100 nucleotides in length, such as e.g. at least 110 nucleotides in length, e.g. at least 120 nucleotides in length, e.g. at least 130 nucleotides in length, such as at least 140 nucleotides in length and most preferably at least 150 nucleotides in length. It is further preferred that the ODN in accordance with the present invention is less than 500 nucleotides in length, such as e.g. less than 400 nucleotides in length, e.g. less than 300 nucleotides in length and most preferably less than 200 nucleotides in length.

Moreover, the oligodesoxynucleotide in accordance with this preferred embodiment is a single-strand ODN (ssODN), i.e. it is not hybridised with a second, different (i.e. complementary or partially complementary) oligonucleotide strand. Nonetheless, it will be appreciated that the ssODN may fold back onto itself, thus forming a partial or complete double stranded molecule consisting of one oligodesoxynucleotide strand. Preferably, the ssODN in accordance with this preferred embodiment does not fold back to form a partial or complete double stranded molecule but instead is single-stranded over its entire length

In a further preferred embodiment, the cells are analysed for successful modification of the target genome.

Methods for analysing for the presence or absence of a modification are well known in the art and include, without being limiting, assays based on physical separation of nucleic acid molecules, sequencing assays as well as cleavage and digestion assays and DNA analysis by the polymerase chain reaction (PCR).

Examples for assays based on physical separation of nucleic acid molecules include without limitation MALDI-TOF, denaturating gradient gel electrophoresis and other such methods known in the art, see for example (Petersen et al., 2002)(Hsia et al., 2005)(Tost and Gut, 2005)(Palais et al., 2005).

Examples for sequencing assays comprise, without limitation, approaches of sequence analysis by direct sequencing, fluorescent SSCP in an automated DNA sequencer and Pyrosequencing. These procedures are common in the art, see e.g. (Adams, 1994) (Alphey, 1997) (Ramon et al., 2003) (Meng et al., 2005).

Examples for cleavage and digestion assays include, without limitation, restriction digestion assays such as restriction fragments length polymorphism assays (RFLP assays), RNase protection assays, assays based on chemical cleavage methods and enzyme mismatch cleavage assays, see e.g.(Youil et al., 1995) (Todd et al., 2001) (Amar et al., 2002).

Alternatively, instead of analysing the cells for the presence or absence of the desired modification, successfully modified cells may be selected by incorporation of appropriate selection markers. Selection markers include positive and negative selection markers, which are well known in the art and routinely employed by the skilled person. Non-limiting examples of selection markers include dhfr, gpt, neomycin, hygromycin, dihydrofolate reductase, G418 or glutamine synthase (GS) (Bebbington et al., 1992). Using these markers, the cells are grown in selective medium and the cells with the highest resistance are selected. Also envisaged are combined positive-negative selection markers, which may be incorporated into the target genome by homologous recombination or random integration. After positive selection, the first cassette comprising the positive selection marker flanked by recombinase recognition sites is exchanged by recombinase mediated cassette exchange against a second, marker-less cassette. Clones containing the desired exchange cassette are then obtained by negative selection.

In a further preferred embodiment of the method of the present invention, the cell is selected from the group consisting of a mammalian or vertebrate cell, and a fungal cell.

In a further preferred embodiment of the method of the invention, the cell is an oocyte.

As used herein the term "oocyte" refers to the female germ cell involved in reproduction, i.e. the ovum or egg cell. In accordance with the present invention, the term "oocyte" comprises both oocytes before fertilisation as well as fertilised oocytes, which are also called zygotes. The oocyte before fertilisation comprises only maternal chromosomes, whereas an oocyte after fertilisation comprises both maternal and paternal chromosomes. After fertilisation, the oocyte remains in a double-haploid status for several hours, in mice for example for up to 18 hours after fertilisation.

In a more preferred embodiment of the method of the invention, the oocyte is a fertilised oocyte.

The term "fertilised oocyte", as used herein, refers to an oocyte after fusion with the fertilizing sperm. For a period of many hours (such as up to 18 hours in mice) after fertilisation, the oocyte is in a double-haploid state, comprising one maternal haploid pronucleus and one paternal haploid pronucleus. After migration of the two pronuclei together, their membranes break down, and the two genomes condense into chromosomes, thereby reconstituting a diploid organism. Preferably, the oocyte used in the method of the present invention is a fertilised oocyte in the double-haploid state.

In one embodiment, the oocyte is not a human oocyte. In another embodiment, the fertilized oocyte is not a human oocyte.

In another preferred embodiment of the method of the invention, the fusion protein or the nucleic acid molecule encoding the fusion protein is introduced into the cell by microinjection. In a further preferred embodiment of the method of the invention, the nucleic acid molecule of step (b) is introduced into the cell by microinjection.

Microinjection into the cell can be carried out by injection into the nucleus and/or by injection into the cytoplasm.
Where the cell is an oocyte, microinjection can be carried out by injection into the nucleus (before fertilisation), the maternal and/or paternal pronucleus (after fertilisation) and/or by injection into the cytoplasm (both before and after fertilisation). When a fertilised oocyte is employed, injection into the pronucleus is carried out either for one pronucleus or for both pronuclei. Preferably, for injection into only one of the pronuclei, the paternal pronucleus is chosen due to its bigger size.
Injection of the fusion protein, of an mRNA or of a DNA encoding the fusion protein in accordance with step (a) of the method of the present invention is preferably into the nucleus/pronucleus, in the case of fertilized oocytes preferably into both pronuclei. It will be appreciated that in the case of mRNA or DNA, it is preferred that said nucleic acid sequence not only encodes the fusion protein, but also includes a nuclear localisation signal to ensure delivery into the nucleus/pronucleus after translation into the fusion protein.

Injection of the nucleic acid molecule of step (b) is preferably into the nucleus/pronucleus, in the case of fertilized oocytes preferably into both pronuclei. However, injection of the nucleic acid molecule of step (b) can also be carried out into the cytoplasm when said nucleic acid molecule is provided as a nucleic acid sequence including a nuclear localisation signal to ensure delivery into the nucleus/pronucleus. Preferably, the microinjection is carried out by injection into both the nucleus/pronucleus and the cytoplasm. For example, the needle can be introduced into the nucleus/pronucleus and a first amount of the fusion protein and/or nucleic acid molecule is injected into the nucleus/pronucleus. While removing the needle from the cell, a second amount of the fusion protein and/or nucleic acid molecule is injected into the cytoplasm.

Methods for carrying out microinjection are well known in the art and are described for example in Nagy et al. (Nagy et al., 2003) as well as in the examples herein below.

In another preferred embodiment of the method of the invention, the nucleic acid molecule encoding the fusion protein in expressible form is mRNA.

In another preferred embodiment of the method of the invention, the regions homologous to the target sequence are localised at the 5' and 3' ends of the donor nucleic acid sequence.

In this preferred embodiment, the donor nucleic acid sequence is flanked by the two regions homologous to the target sequence such that the nucleic acid molecule used in the method of the present invention consists of a first region homologous to the target sequence, followed by the donor nucleic acid sequence and then a second region homologous to the target sequence.

In a further preferred embodiment of the method of the invention, the regions homologous to the target sequence comprised in the nucleic acid molecule of (b) have a length of at least 400 bp. More preferably, the regions each have a length of at least 500 nucleotides, such as at least 600 nucleotides, at least 750 bp nucleotides, more preferably at least 1000 nucleotides, such as at least 1500 nucleotides, even more preferably at least 2000 nucleotides and most preferably at least 2500 nucleotides. It will be appreciated that these minimum lengths refer to the lengths of each of the homologous regions present in the nucleic acid molecule of (b), i.e. where two homologous regions are present, each homologous independently has a length of at least 400bp, 500bp etc., wherein the homologous regions may have the same or different lengths, as long as they each have the recited minimum length. The maximum length of the regions homologous to the target sequence comprised in the nucleic acid molecule depends on the type of cloning vector used and can usually be up to a length 20.000 nucleotides each in E. coli high copy plasmids using the col EI replication origin (e.g. pBluescript) or up to a length of 300.000 nucleotides each in plasmids using the F-factor origin (e.g. in BAC vectors such as for example pTARBAC1).

In a further preferred embodiment of the method of the invention, the modification of the target sequence is selected from the group consisting of substitution, insertion and deletion of at least one nucleotide of the target sequence. Preferred in accordance with the present invention are substitutions, for example substitutions of 1 to 3 nucleotides and insertions of exogenous sequences, such as IoxP sites (34 nucleotides long) or cDNAs, such as for example for reporter genes. Such cDNAs for reporter genes can, for example, be up to 6 kb long. Depending on the purpose of the modification, the modifications should be in frame or should lead to a frame shift. The person skilled in the art knows how to ensure that the reading frame is maintained or shifted and would also be aware which alternative is desirable in a particular case.

In another preferred embodiment of the method of the invention, the cell is from a mammal selected from the group consisting of rodents, dogs, felides, primates, rabbits, pigs, and ruminants or wherein the cell is from an avian selected from the group consisting of chickens, turkeys, pheasants, ducks, geese, quails and ratites including ostriches, emus and cassowaries or wherein the cell is from zebrafish.

All of the mammals, avians and fish described herein are well known to the skilled person and are taxonomically defined in accordance with the prior art and the common general knowledge of the skilled person.

Non-limiting examples of "rodents" are mice, rats, squirrels, chipmunks, gophers, porcupines, beavers, hamsters, gerbils, guinea pigs, degus, chinchillas, prairie dogs, and groundhogs.

Non-limiting examples of "dogs" include members of the subspecies canis *lupus familiaris* as well as wolves, foxes, jackals, and coyotes.

Non-limiting examples of "felides" include members of the two subfamilies: the pantherinae, including lions, tigers, jaguars and leopards and the felinae, including cougars, cheetahs, servals, lynxes, caracals, ocelots and domestic cats.

The term "primates", as used herein, refers to all monkey including for example cercopithecoid (old world monkey) or platyrrhine (new world monkey) as well as lemurs, tarsiers, apes and marmosets (Callithrix jacchus). In one embodiment, the mammal is a non-human mammal.
Examples of "ruminants" include, without being limiting, cattle, goats, sheep, giraffes, bisons, mooses, elks, yaks, water buffalos, deer, camels, alpacas, Ilamas, antelopes, pronghorns, and nilgais. Preferably, the ruminants are selected from the group consisting of cattle, goats and sheep.

The present invention also relates to a method of producing a non-human vertebrate or mammal carrying a modified target sequence in its genome, the method comprising the steps of: (a) producing a cell in accordance with the method of the invention; (b) transferring the cell obtained in (a) into a pseudo pregnant female host; and (c) analysing the offspring delivered by the female host for the presence of the modification.

In accordance with the present invention, the term "transferring the oocyte obtained in (a) to a pseudopregnant female host" includes the transfer of a fertilised oocyte but also the transfer of pre-implantation embryos of for example the 2-cell, 4-cell, 8-cell, 16-cell and blastocyst (70- to 100-cell) stage. Said pre-implantation embryos can be obtained by culturing the oocyte under appropriate conditions for it to develop into a pre-implantation embryo. Furthermore, the oocyte may be injected into a blastocyst or fused with a blastocyst in order to obtaining a pre-implantation embryo. Methods of introducing an oocyte into a blastocyst as well as methods for transferring an oocyte or pre-implantation embryo to a pseudo-pregnant female host are well known in the art and are, for example, described in Nagy et al., (Nagy et al., 2003).

It is further envisaged in accordance with the method of producing a non-human vertebrate or mammal carrying a modified target sequence in its genome that a step of analysis of successful genomic modification is carried out before transplantation into the female host. As a non-limiting example, the oocyte can be cultured to the 2-cell, 4-cell or 8-cell stage and one cell can be removed without destroying or altering the resulting embryo. Analysis for the genomic constitution, e.g. the presence or absence of the genomic modification, can then be carried out using for example PCR or southern blotting techniques or any of the methods described herein above. Such methods of analysis of successful genotyping prior to transplantation are known in the art and are described, for example in Peippo et al.(Peippo et al., 2007).
For this method of producing a non-human vertebrate or mammal, fertilisation of the oocyte is required. Said fertilisation can occur before the modification of the target sequence in step (a) in accordance with the method of producing a non-human vertebrate or mammal of the invention, i.e. a fertilised oocyte can be used for the method of modifying a target sequence in accordance with the invention. The fertilisation can also be carried out after the modification of the target sequence in step (a), i.e. a non-fertilised oocyte can be used for the method of modifying a target sequence in accordance with the invention, wherein the oocyte is subsequently fertilised before transfer into the pseudopregnant female host.

The step of analysing for the presence of the modification in the offspring delivered by the female host provides the necessary information whether or not the produced non-human vertebrate or mammal carries the modified target sequence in its genome. Thus, the presence of the modification is indicative of said offspring carrying a modified target sequence in its genome whereas the absence of the modification is indicative of said offspring not carrying the modified target sequence in its genome. Methods for analysing for the presence or absence of a modification have been detailed above. Those offspring carrying the modified target sequence in their genome can then be further bred in order to determine whether the introduced modification is passed on to offspring via germline transmission. Those mammals in which germline transmission of the modification is successful can then be used for further breeding.

The non-human vertebrate or mammal produced by the method of the invention is, inter alia, useful to study the function of genes of interest and the phenotypic expression/outcome of modifications of the genome in such animals. It is furthermore envisaged that the non-human vertebrates or mammals of the invention can be employed as disease models for diseases such as human familial amyotrophic lateral sclerosis, frontotemporal demential, Parkinson's disease, Alzheimer's disease and any other genetically caused diseases and for testing therapeutic agents/compositions. Furthermore, the non-human vertebrate or mammal of the invention can also be used for livestock breeding.

Preferably, this method of the invention of producing a non-human vertebrate or mammal carrying a modified target sequence in its genome further comprises culturing the cell to form a pre-implantation embryo or introducing the cell into a blastocyst prior to transferring it into the pseudopregnant female host.

Methods for culturing the cell to form a pre-implantation embryo or introducing the cell into a blastocyst are well known in the art and are, for example, described in Nagy *et al.,* loc. cit. The term "introducing the cell into a blastocyst" as used herein encompasses injection of the cell into a blastocyst as well as fusion of a cell with a blastocyst. Methods of introducing a cell into a blastocyst are described in the art, for example in Nagy *et al.,* loc. cit..

In a preferred embodiment, the non-human mammal is selected from the group consisting of rodents, dogs, felides, primates, rabbits, pigs, and ruminants or the vertebrate is selected from the group consisting of fish, such as for example zebrafish, salmon, trout, common carp or coi carp, or from avians, such as for example chickens, turkeys, pheasants, ducks, geese, quails and ratites including ostriches, emus and cassowaries.

The present invention further relates to a non-human vertebrate or mammalian animal obtainable by the above described method of the invention.

All the definitions and preferred embodiments defined above with regard to the method of the invention of producing a non-human, mammalian oocyte carrying a modified target sequence in its genome apply *mutatis mutandis* also to this method of the invention of producing a non-human vertebrate or mammalian.

The present invention further relates to a fusion protein comprising a DNA-binding domain of a RTL effector protein and a non-specific cleavage domain of a restriction nuclease. All the definitions and preferred embodiments defined above with regard to the fusion protein in the context of the methods of the invention apply *mutatis mutandis.* Furthermore, the present invention also relates to a kit comprising the fusion protein of the invention. The various components of the kit may be packaged in one or more containers such as one or more vials. The vials may, in addition to the components, comprise preservatives or buffers for storage. In addition, the kit may contain instructions for use.

The figures show:

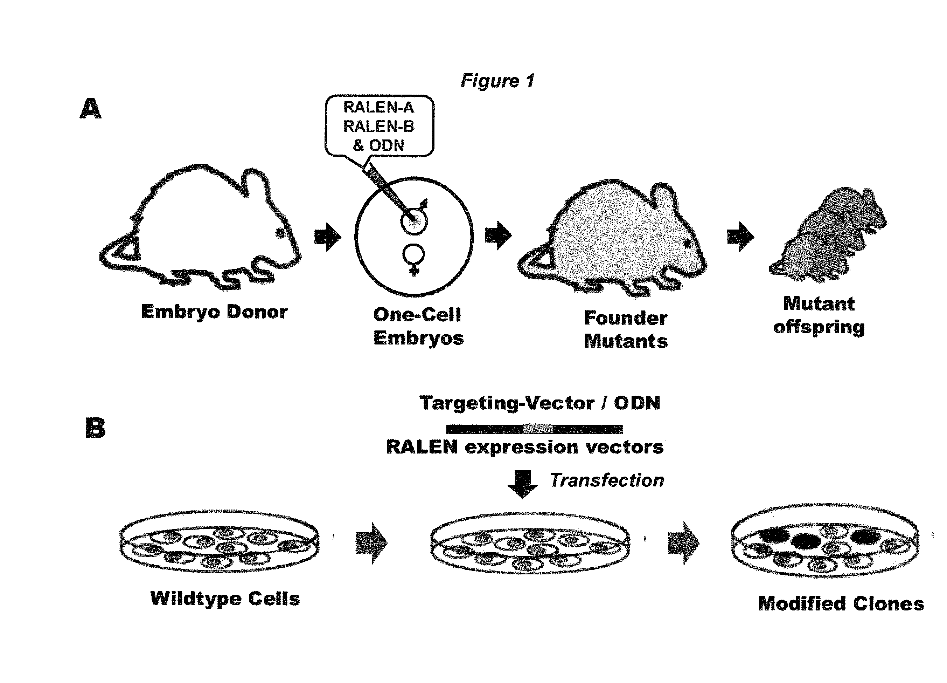

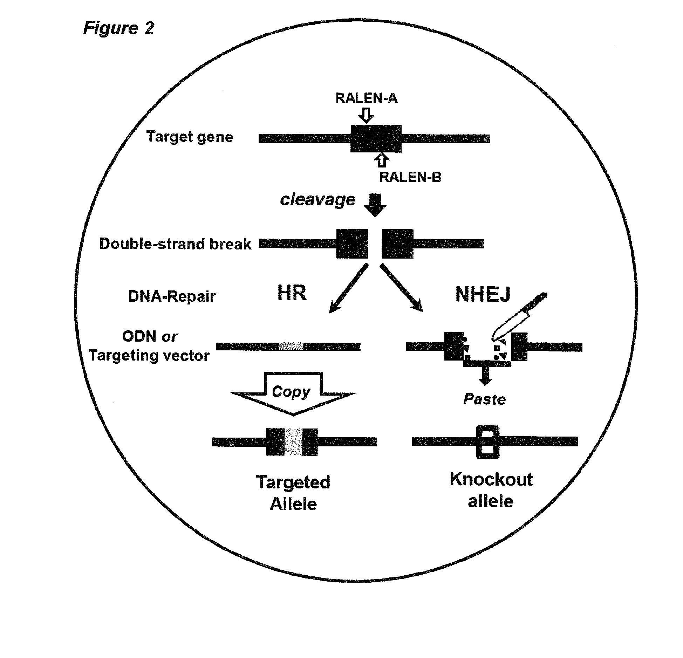

**Figure 1****: Schematic outline of RALEN-mediated genome modifications. A:** The workflow of RALEN-mediated, embryo based gene targeting is shown which starts with the microinjection of RALEN-A and -B mRNAs (or proteins) and optionally, of a synthetic, mutagenic oligodeoxynucleotide (ODN), into one or both pronuclei of one-cell embryos isolated from donor females. Upon translation, the RALEN proteins are imported into the pronuclei and create a double strand break (DSB) in the target gene of the paternal and maternal genome (see Fig.2). The repair of DSBs by NHEJ or by homologous recombination creates a modification within the target gene. Upon the transfer of microinjected embryos into foster females, the offspring derived is genotyped by PCR as well as sequence analysis to identify founder animals that harbor targeted or knockout mutations in their germline. The mating of such founders to wildtype mice produces heterozygous mutants that enable to establish mutant breeding colonies. B: RALEN-mediated genome modification in mammalian cell lines. Cells are transfected with RALEN expression vectors together with or without a targeting vector or ODN. From the transfected population subclones are established and genotyped for the presence of modifications within the target gene (black cells).
**Figure 2****: RALEN mediated gene editing in mammalian cells and embryos.** Double-strand breaks (DSBs) induced by RALEN-A and -B enhance DNA repair at the target site by several orders of magnitude. DSBs may be repaired by the homologous recombination (HR) pathway using a synthetic oligonucleotide or a gene targeting vector as repair template, that contain a desired genetic modification flanked with sequence homology regions. In the recombination process, gene conversion extends from the vector's homology regions into the heterologous sequence and transfers the modification into the genome (targeted allele). Alternatively, DSBs can be closed by the non-homologous end joining (NHEJ) pathway that re-ligates the open DNA ends without repair templates. By this means, DNA ends are frequently edited through loss of multiple nucleotides causing in many cases frameshift (knockout) mutations within coding regions.

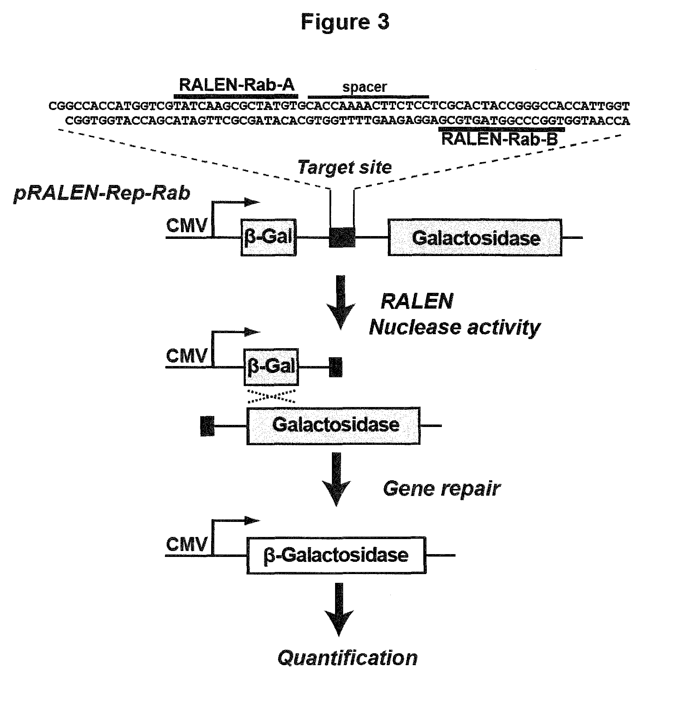

**Figure 3****: RALEN nuclease reporter assay in 293 cells.** The nuclease reporter plasmid pRALEN-Rep contains a CMV promoter region, a 400 bp sequence coding for the N-terminal segment of β-galactosidase and a stop codon. This unit is followed by a target sequence derived from exon 1 of the mouse Rab38 gene. The 15 bp recognition sequences of the RALEN-A and -B proteins are indicated and separated by a 17 bp spacer region. The RALEN target sequence is followed by the complete coding region for β-galactosidase (except the start codon) and a polyadenylation signal. To test for nuclease activity against the target sequence, RALEN nuclease expression vectors are transfected into HEK293 cells. Upon expression of the nuclease proteins the reporter plasmid is opened by a nuclease induced double strand-break within the target sequence. The DNA regions adjacent to the double-strand break are identical over 400 bp and can be aligned and recombined (X) by homologous recombination DNA repair. Homologous recombination of an opened reporter plasmid results into a functional β-galactosidase coding region that produces β-galactosidase enzyme. After two days the transfected cell population is lysed and the enzyme activity quantified by a chemiluminescense assay.

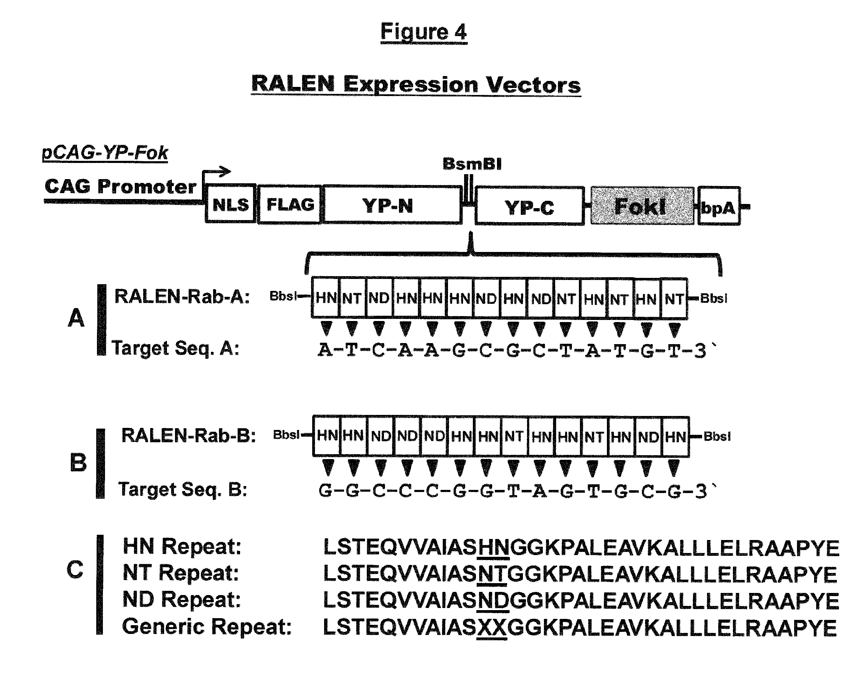

**Figure 4****: DNA constructs of the invention for the production of RALEN proteins.**
   The RALEN expression vector pCAG-PY-Fok contains a CAG promoter region and a transcriptional unit comprising an ATG start codon (arrow), a nuclear localisation sequence (NLS), a FLAG Tag sequence (FLAG), a linker sequence, a segment coding for the N-terminal domain (YP-N) of the Ralstonia protein YP003752492.1, located upstream of a central pair of BsmBI restriction sites. Downstream of the BsmBI sites the transcriptional unit contains C-terminal sequences (YP-C) derived from the Ralstonia protein YP003752492.1, the coding sequence of the FokI nuclease domain and a polyadenylation signal sequence (bpA). DNA segments coding for DNA binding RALEN domains by the fusion of RTL repeats can be inserted into the BsmBI sites of pCAG-PY-Fok for the expression of variable RALEN-Fok nuclease fusion proteins. A: to create a RALEN-Rab-A coding vector an array of 14 RTL repeats, recognising the target sequence Seq.A from the Rab38 gene, was inserted into pCAG-PY-Fok to derive the expression vector pCAG-PY-RALEN-RabA-Fok. Amino acid diresidues within boxes represent positions 12 and 13 of RTL repeats used to recognize the respective nucleotide of the target sequence. **B:** to create a RALEN-Rab-B coding vector an array of 14 RTL repeats, recognising the target sequence Seq.B from the Rab38 gene, was inserted into pCAG-PY-Fok to derive the expression vector pCAG-PY-RALEN-RabB-Fok. Amino acid diresidues within boxes represent positions 12 and 13 of the RTL repeats used to recognize the respective nucleotide of the target sequence. **C**: Sequence of 35 amino acids RTL repeats used in A) and B) for the constriction of RALEN coding regions. The repeat's amino acid code at positions 12/13 confers binding to one of the DNA nucleotides of the target sequence (HN > A or G, NT > T, ND > C), in the generic RTL repeat sequence the positions are filled by XX.

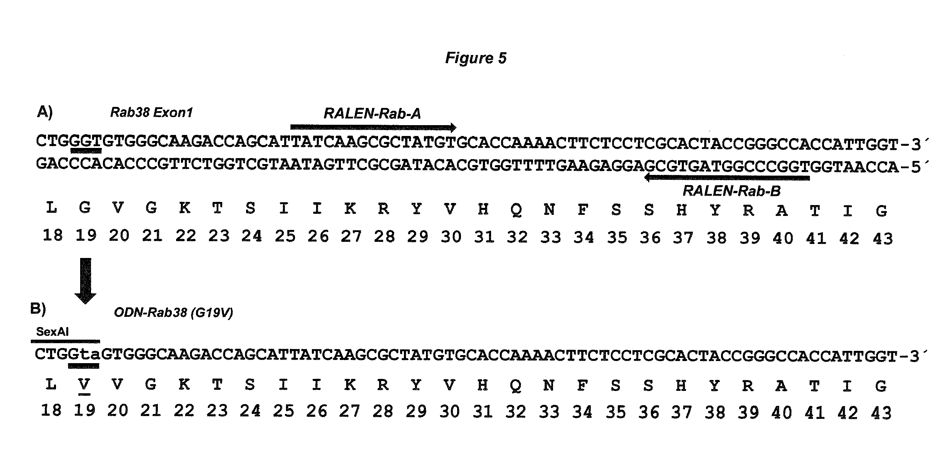

**Figure 5****: RALEN-mediated targeted mutation of the murine *Rab38* gene. A:** Codons 18 - 43 within exon 1 of the mouse *Rab38* gene. Using the mutagenic oligodeoxynucleotide ODN-Rab38 (G19V) as repair template for RALEN-Rab-A and -B (Fig. 4) induced double-strand breaks within the indicated target region in Neuro2A cells or one-cell mouse embryos, a glycine to valine replacement (small letters) and a SexAI site is created at codon 19 (underlined).

The examples illustrate the invention.

### Example 1: Generation of knockout and knockin mutations in the Rab38 gene by RALENs in mouse cells and one-cell embryos.

As proof of RALEN-induced mutagenesis, the *Rab38* gene is targeted to create a glycine-to-valine missense mutation at codon 19 (G19V) (Fig. 5) within the first exon (SEQ ID NO:24), as found in the *Rab38^{cht}* allele of chocolate mutants (Loftus et al., 2002). The *Rab38* gene encodes a small GTPase that regulates intracellular vesicle trafficking in melanocytes, retinal pigment epithelial cells, alveolar pneumocytes and platelets (Wasmeier et al., 2006). Mutant *chocolate* mice (*Rab38^{cht}*) exhibit a missense and *ruby* rats a nonsense mutation within *Rab38* and are considered to be phenotypic models of Hermansky-Pudlak syndrome; a disease characterized by oculocutaneous albinism, progressive pulmonary fibrosis and platelet storage disease (Oiso et al., 2004)(Di Pietro and Dell'Angelica, 2005)(Lopes et al., 2007)(Osanai et al., 2010). As targeting molecule a synthetic, single-stranded oligodeoxynucleotide ODN-Rab38(G19V) of 144 nucleotides (SEQ ID NO:1) is used that covers 47 bp of the lagging strand sequence upstream of codon 19 and 94 bp of downstream sequence. ODN-Rab38 (G19V) includes a G to T replacement at the second position of codon 19, creating a valine triplet and a SexAI restriction site, and a silent T to A exchange as an unique identifier of the targeted *Rab38* allele (Fig. 5).

For the targeting of *Rab38* two *Ralstonia* effector nuclease (RALEN) DNA binding domains are designed that are able to recognize sequences within exon 1 (Fig. 3, Fig. 4) (SEQ ID NO:24), located downstream of codon 19 (Fig. 5). By the combination of RTL repeats, derived from *Ralstonia* effector proteins, containing appropriate diresidues at position 12 and 13 (Fig. 4), specifically using HN to recognize A and G nucleotides (SEQ ID NO:2) using NT for recognition of T (SEQ ID NO:3) and of ND to recognize C (SEQ ID NO:4) within the backbone of the repeat comprising the residues 240-274 of the Ralstonia effector protein YP003752492.1 (SEQ ID NO:5), the RALEN-Rab-A (SEQ ID NO:6) (Fig. 4A) and RALEN-Rab-B (SEQ ID NO:7) (Fig. 4B) DNA binding domains are obtained, encoded by synthetic gene fragments RALEN-Rab-A (SEQ ID NO:8) and RALEN-Rab-B (SEQ ID NO:9) that include Bbsl recognition sites at both ends of the coding region. The RALEN coding regions are digested with Bbsl and ligated into the expression vector backbone of pCAG-PY-Fok (SEQ ID NO:11), opened with BsmBI (Fig. 4). The vector backbone provides a CAG promoter for expression in mammalian cells, a T7 promoter for in vitro production of mRNA, a Flag Tag and nuclear localization (NLS) sequence, a C-terminal FokI nuclease domain and sequences from the *Ralstonia* effector protein YP003752492.1 (SEQ ID NO:5), flanking the RALEN domains at the N- and C-terminal ends (Fig. 4). By ligation of the RALEN coding regions into the BsmBI sites of pCAG-PY-Fok we obtain the pair of RALEN expression vectors pCAG-PY-RALEN-RabA-Fok (SEQ ID NO:16) and pCAG-PY-RALEN-RabB-Fok (SEQ ID NO:17) coding for the RALEN-Fok fusion proteins PY-RALEN-RabA-Fok (SEQ ID NO:18) and PY-RALEN-RabB-Fok (SEQ ID NO:19).

In order to test for the specific nuclease activity of the RALEN-Fok fusion proteins the nuclease reporter plasmid pRALEN-Rep-Rab (SEQ ID NO:20) is used, containing the RALEN target sequences in between a partially duplicated β**-**Galactosidase reporter gene (Fig. 3). To test for nuclease activity, the reporter plasmid is cotransfected with plasmids pCAG-PY-RALEN-RabA-Fok and pCAG-PY-RALEN-RabB-Fok into human HEK293 cells. Upon expression of the nuclease proteins the reporter plasmid is opened by a nuclease induced double strand-break within the target sequence. The DNA regions adjacent to the double-strand break are identical over 400 bp and can be aligned and recombined by homologous recombination DNA repair. Homologous recombination of an opened reporter plasmid results into a functional β-galactosidase coding region that produces β-galactosidase enzyme (Fig. 3). After two days the transfected cell population is lysed and the enzyme activity quantified by a chemiluminescense assay in comparison to samples that are transfected with the reporter plasmid alone.

Next, we validate the utility of our RALEN-Fok fusion proteins to modify the genomic Rab38 gene in the mouse cell line NEURO-2A. For this purpose the expression vectors pCAG-PY-RALEN-RabA-Fok and pCAG-PY-RALEN-RabB-Fok, are cotransfected with ODN-Rab38(G19V) into NEURO-2A cells. Two days after transfection the cells are seeded at low density and sublones are isolated and genotyped for the presence of mutations within the RALEN target region.

Next we assess the utility of the derived RALEN proteins for inducing mutations in the genome of mouse one-cell embryos. The workflow of embryo based gene targeting starts with the microinjection of RALEN mRNAs or proteins and optional, of a synthetic oligodeoxynucleotide (ODN), into the paternal pronucleus of one-cell embryos isolated from donor females. Upon translation, the RALEN nuclease proteins are imported into the pronuclei and create a double strand break in the target gene of the paternal and maternal genome (Fig.1). In the paternal genome, DSBs are sealed either by homologous recombination with the mutagenic ODN or become processed in both genomes by error-prone NHEJ repair, creating knockin or knockout alleles (Fig. 2). Upon transfer of the microinjected embryos into foster females, the offspring derived is genotyped by PCR and sequence analysis to identify founder animals that harbor targeted or knockout mutations in their germline. The mating of such founders to wildtype mice produces heterozygous mutants that are intercrossed to obtain homozygote mutants. ODN-Rab38 (G19V) is microinjected together with mRNAs coding for the PY-RALEN-RabA-Fok and PY-RALEN-RabB-Fok, that are produced by T7 polymerase driven in vitro transcription from the respective expression vectors. The resulting mice are analysed for gene editing events by PCR amplification of a 213 bp region covering the first exon of *Rab38* (SEQ ID NO:24) from genomic tail DNA. Founder mice harboring the G19V replacement are initially identified by the digestion of PCR products with SexAl. The presence of digested PCR products identifies a substantial fraction of the pups derived from microinjections of PY-RALEN-RabA-Fok and PY-RALEN-RabB-Fok mRNAs as recombined founders, indicating the activity of RALENs in mammalian embryos. Subsequently, undigested PCR products from such founders are subcloned and individual subclones are analysed by sequencing. This analysis reveals the presence of Rab38 alleles harboring the G19V replacement but also of knockout alleles that lost a variable number of nucleotides due to NHEJ repair. By further breeding of such founder mice the mutant Rab38 alleles can be transferred via the germ line and enable the establishment of mutant mouse lines.

### Methods

### Vector constructions

For the expression of RALEN nucleases in mammalian cells we construct the generic expression vector pCAG pCAG-PY-Fok (Seq ID NO: 11) (Fig. 4), that contains a CAG hybrid promoter region and a transcriptional unit comprising a sequence coding for an ATG start codon, a nuclear localisation sequence, a FLAG Tag sequence, a linker sequence, the amino acids 1 - 204 of the *Ralstonia* effector protein YP003752492.1 (SEQ ID NO:5), located upstream of a pair of BsmBI restriction sites. Downstream of the central BsmBI sites, the transcriptional unit contains 79 codons (590 -668) of the Ralstonia effector protein YP003752492.1 (SEQ ID NO:5), followed by the coding sequence of the FokI nuclease domain (Seq ID NO:21) and a polyadenylation signal sequence (bpA). DNA segments coding for RALEN arrays of RTL repeats, designed to bind target sequences within the Rab38 gene (Fig. 4), are obtained by gene synthesis (Genscript, Piscataway, USA), digested with BbsI and ligated into the BsmBI sites of pCAG-PY-Fok to obtain the RALEN expression vector pair pCAG-PY-RALEN-RabA-Fok (SEQ ID NO:16) and pCAG-PY-RALEN-RabB-Fok (SEQ ID NO:17).

The RALEN nuclease reporter plasmid pRALEN-Rep-Rab (SEQ ID NO:20) (Fig. 3) contains a CMV promoter region, a 400 bp sequence coding for the N-terminal segment of β-galactosidase, a stop codon, the nuclease target region derived from the mouse Rab38 gene and the coding region for β-galactosidase (except the start codon) and a polyadenylation signal (pA). To test for nuclease activity against the specific target sequence RALEN expression vectors are transiently cotransfected with the reporter plasmid into HEK293 cells. Upon expression of the nuclease protein the reporter plasmid is opened by a nuclease-induced double-strand break within the target sequence (Fig. 3). The DNA regions adjacent to the double-strand break are identical over 400 bp and can be aligned and recombined by homologous recombination DNA repair. Recombination of an opened reporter plasmid will subsequently result into a functional β-galactosidase coding region transcribed from the CMV promoter that leads to the production of β-galactosidase protein. In lysates of transfected cells the enzymatic activity of β-galactosidase can be determined by chemiluminescense.

### Nuclease activity in mammalian cell lines

To demonstrate the activity of RALENs in mammalian cells, we electroporate one million HEK 293 cells (ATCC #CRL-1573) (Graham et al., 1977) with 5 µg plasmid DNA each of one of the RALEN nuclease expression vectors together with 5 µg of the reporter plasmid. In addition, each sample receives 5 µg of the firefly Luciferase expression plasmid pCMV-hLuc and is adjusted to a total DNA amount of 20 µg with pBluescript (pBS) plasmid DNA. Upon transfection the cells are seeded in triplicate wells of a 6-well tissue culture plate and cultured for two days before analysis is started. For analysis the transfected cells of each well are lysed and the β-galactosidase and luciferase enzyme activities of the lysates are individually determined using chemiluminescent reporter assays following the manufacturer's instruction (Roche Applied Science, Germany) in a luminometer (Berthold Centro LB 960). As positive control we transfect 5 µg of the β-galactosidase expression plasmid pCMVβ with 15 µg pBS, as negative control 5 µg pCMV-hLuc are transfected with 15 µg pBS. The triplicate β-galactosidase values of each sample are normalised in relation to the levels of Luciferase activity and the mean value and standard deviation of β-galactosidase activity are calculated and expressed in comparison to the pCMVβ positive control defined as 1.0. In this type of recombination assay the level of the β-galactosidase catalysed light emission reflects the cleavage and repair of the reporter plasmids and thereby indicates the activity of RALEN nucleases.

To demonstrate the activity of Rab38 specific RALENs on genomic DNA of mouse cells (Fig. 1B), we electroporate one million Neuro-2A cells (ATCC #CCL-131) (Klebe and Ruddle, 1969) with 5 µg plasmid DNA each of one of the RALEN nuclease expression vector pairs together with 5 µg of the synthetic oligodeoxynucleotide ODN-Rab38(G19V (SEQ ID NO:1). Two days after electroporation the cells are seeded at low density and sublones are isolated and genotyped for the presence of mutations within the Rab38 target region. Genomic DNA is isolated from NEURO-2A subclones, following the Wizard Genomic DNA Purification Kit (Promega) protocol. The resulting DNA pellets are dissolved in 100 µl 10 mM Tris-CI, pH 8.5, incubated over night at room temperature and stored for further analysis at 4 °C. To analyze subclones for mutations in the Rab38 gene (Fig. 5), exon 1 (SEQ ID NO:24) is amplified using the PCR primer pair Rab-for (SEQ ID NO:22) (5'-GGCCTCCAGGATGCAGACACC-3') and Rab-rev (SEQ ID NO:23) (5'-CCAGCAATGTCCCAGAGCTGC-3'). Amplification is performed using Herculase II polymerase (Agilent Technologies) in 25 µl reactions with 30 cycles of 95 °C - 20 s, 60 °C - 15 s, 72 °C - 15 s. Afterwards, the PCR products are directly digested with 10 U of SexAI and analyzed on agarose gels. Undigested products from positively identified founders are purified with the Qiaquick PCR purification Kit (Qiagen), cloned into pSC-B (Stratagene, La Jolla, USA) and sequenced. The results are compared to the genomic Rab38 sequences using the Vector NTI software (Invitrogen).

### Microinjection of mouse one-cell embryos

The injection of RALEN mRNAs and the targeting ODN (SEQ ID NO:1) (Fig. 1A) is performed as previously described for ZFNs (Meyer et al., 2010)(Meyer et al., 2012). Briefly, mRNA coding for PY-RALEN-RabA-Fok (SEQ ID NO:18) and PY-RALEN-RabB-Fok (SEQ ID NO:19) is prepared by *in vitro* transcription and polyadenylation from plasmid DNAs, linearized at the end of the transcribed region with Mlul, using the mMessage mMachine T7 Ultra kit and the MEGAclear kit (Life Technologies, Carlsbad, USA). Each of the mRNAs is then diluted into injection buffer (10mM Tris, 0.1 mM EDTA, pH7.2) to a working concentration of 20 ng/µl. The targeting oligodeoxynucleotide (Metabion, Martinsried, Germany) is diluted in injection buffer to a working concentration of 30 ng/µl. Appropriate mRNAs pairs are mixed with the respective mutagenic oligodesoxynucleotide and stored at -80 °C. One-cell embryos are obtained by mating of C57BL/6N males with super-ovulated FVB females (Charles River, Sulzbach, Germany). For super-ovulation three-week old FVB females are treated with 2.5 IU pregnant mares serum (PMS) 2 days before mating and with 2.5 IU Human chorionic gonadotropin (hCG) at the day of mating. Fertilised oocytes are isolated from the oviducts of plug positive females and microinjected in M2 medium (Sigma-Aldrich Inc Cat. No. M7167) following standard procedures (Nagy A, Gertsenstein M, Vintersten K, Behringer R., 2003. Manipulating the Mouse Embryo. Cold Spring Harbour, New York: Cold Spring Harbour Laboratory Press). Embryos are injected with the mixture of the targeting ODN and the mRNAs in a two-step procedure, as described (Meyer et al. (2010) Proc Natl Acad Sci U S A 107:15022-6; Meyer et al. (2012) Proc Natl Acad Sci USA 109:9354-9359). Briefly, a first aliquot of the DNA/RNA mixture is injected into, whenever possible, the larger (male) pronucleus to deliver the DNA vector, as used for the production of transgenic mice. Upon the withdrawal of the injection needle from the pronucleus a second aliquot of the DNA/RNA mixture is injected into the cytoplasm to deliver the mRNAs directly to the translation machinery. Injections are performed using a Leica micromanipulator and microscope and an Eppendorf FemtoJet injection device. Injected zygotes are transferred into pseudopregnant CD1 female mice and viable adult mice were obtained.

### Genotying of founder mice

Genomic DNA is isolated from tail tips of mice derived from microinjections, following the Wizard Genomic DNA Purification Kit (Promega) protocol. The resulting DNA pellets are dissolved in 100 µl 10 mM Tris-Cl, pH 8.5, incubated over night at room temperature and stored for further analysis at 4 °C. To analyze founders for mutations in the *Rab38* gene (Fig. 5), exon 1 (SEQ ID NO:24) is amplified using the PCR primer pair Rab-for (SEQ ID NO:22) (5'-GGCCTCCAGGATGCAGACACC-3') and Rab-rev (SEQ ID NO:23) (5'-CCAGCAATGTCCCAGAGCTGC-3'). Amplification is performed using Herculase II polymerase (Agilent Technologies) in 25 µl reactions with 30 cycles of 95 °C - 20 s, 60 °C - 15 s, 72 °C - 15 s. Afterwards, the PCR products are directly digested with 10 U of SexAI and analyzed on agarose gels. Undigested products from positively identified founders are purified with the Qiaquick PCR purification Kit (Qiagen), cloned into pSC-B (Stratagene, La Jolla, USA) and sequenced. The results are compared to the genomic Rab38 sequences using the Vector NTI software (Invitrogen).

### References

Adams (1994). Automated DNA Sequencing and Analysis (Academic Press).
Alphey (1997). DNA Sequencing: From Experimental Methods to Bioinformatics (Springer Verlag).
Altschul, S.F., and Gish, W. (1996). Local alignment statistics. Meth. Enzymol. 266, 460-480.
Altschul, S.F., Gish, W., Miller, W., Myers, E.W., and Lipman, D.J. (1990). Basic local alignment search tool. J. Mol. Biol. 215, 403-410.
Amar, C.F.L., Dear, P.H., Pedraza-Diaz, S., Looker, N., Linnane, E., and McLauchlin, J. (2002). Sensitive PCR-restriction fragment length polymorphism assay for detection and genotyping of Giardia duodenalis in human feces. J. Clin. Microbiol. 40, 446-452.
Bebbington, C.R., Renner, G., Thomson, S., King, D., Abrams, D., and Yarranton, G.T. (1992). High-level expression of a recombinant antibody from myeloma cells using a glutamine synthetase gene as an amplifiable selectable marker. Biotechnology (N.Y.) 10, 169-175.
Bloch, K.D. (2001). Mapping by multiple endonuclease digestions. Curr Protoc Mol Biol Chapter 3, Unit3 2.
Boch, J., Scholze, H., Schornack, S., Landgraf, A., Hahn, S., Kay, S., Lahaye, T., Nickstadt, A., and Bonas, U. (2009). Breaking the code of DNA binding specificity of TAL-type III effectors. Science 326, 1509-1512.
Bradley, A., Evans, M., Kaufman, M.H., and Robertson, E. (1984). Formation of germ-line chimaeras from embryo-derived teratocarcinoma cell lines. Nature 309, 255-256.
Capecchi, M.R. (2005). Gene targeting in mice: functional analysis of the mammalian genome for the twenty-first century. Nat Rev Genet 6, 507-512.
Carbery, I.D., Ji, D., Harrington, A., Brown, V., Weinstein, E.J., Liaw, L., and Cui, X. (2010). Targeted genome modification in mice using zinc-finger nucleases. Genetics 186, 451-459.
Christian, M., Cermak, T., Doyle, E.L., Schmidt, C., Zhang, F., Hummel, A., Bogdanove, A.J., and Voytas, D.F. (2010). Targeting DNA double-strand breaks with TAL effector nucleases. Genetics 186, 757-761.
Collins, F.S., Rossant, J., and Wurst, W. (2007). A mouse for all reasons. Cell 128, 9-13.
Cui, X., Ji, D., Fisher, D.A., Wu, Y., Briner, D.M., and Weinstein, E.J. (2011). Targeted integration in rat and mouse embryos with zinc-finger nucleases. Nat Biotechnol 29, 64-67.
Doyon, Y., McCammon, J.M., Miller, J.C., Faraji, F., Ngo, C., Katibah, G.E., Amora, R., Hocking, T.D., Zhang, L., Rebar, E.J., et al. (2008). Heritable targeted gene disruption in zebrafish using designed zinc-finger nucleases. Nat Biotechnol 26, 702-708.
Evans, M.J., and Kaufman, M.H. (1981). Establishment in culture of pluripotential cells from mouse embryos. Nature 292, 154-156.
Flisikowska, T., Thorey, I.S., Offner, S., Ros, F., Lifke, V., Zeitler, B., Rottmann, O., Vincent, A., Zhang, L., Jenkins, S., et al. (2011). Efficient immunoglobulin gene disruption and targeted replacement in rabbit using zinc finger nucleases. PLoS One 6, e21045.
Genin, S., and Denny, T.P. (2012). Pathogenomics of the Ralstonia solanacearum species complex. Annu Rev Phytopathol 50, 67-89.
Geurts, A.M., Cost, G.J., Freyvert, Y., Zeitler, B., Miller, J.C., Choi, V.M., Jenkins, S.S., Wood, A., Cui, X., Meng, X., et al. (2009). Knockout rats via embryo microinjection of zinc-finger nucleases. Science 325, 433.
Gong, M., and Rong, Y.S. (2003). Targeting multi-cellular organisms. Curr Opin Genet Dev 13, 215-220.
Gossler, A., Doetschman, T., Korn, R., Serfling, E., and Kemler, R. (1986). Transgenesis by means of blastocyst-derived embryonic stem cell lines. Proc. Natl. Acad. Sci. U.S.A. 83, 9065-9069.
Graham, F.L., Smiley, J., Russell, W.C., and Nairn, R. (1977). Characteristics of a human cell line transformed by DNA from human adenovirus type 5. J. Gen. Virol. 36, 59-74.
Heuer, H., Yin, Y.-N., Xue, Q.-Y., Smalla, K., and Guo, J.-H. (2007). Repeat domain diversity of avrBs3-like genes in Ralstonia solanacearum strains and association with host preferences in the field. Appl. Environ. Microbiol. 73, 4379-4384.
Hsia, A.-P., Wen, T.-J., Chen, H.D., Liu, Z., Yandeau-Nelson, M.D., Wei, Y., Guo, L., and Schnable, P.S. (2005). Temperature gradient capillary electrophoresis (TGCE)--a tool for the high-throughput discovery and mapping of SNPs and IDPs. Theor. Appl. Genet. 111, 218-225.
Klebe, and Ruddle (1969). The Journal of Cell Biology 43, 69A.
Klug, A. (2010). The discovery of zinc fingers and their applications in gene regulation and genome manipulation. Annu. Rev. Biochem. 79, 213-231.
Lai, L., and Prather, R.S. (2003). Creating genetically modified pigs by using nuclear transfer. Reprod Biol Endocrinol 1, 82.
Li, L., Atef, A., Piatek, A., Ali, Z., Piatek, M., Aouida, M., Sharakou, A., Mahjoub, A., Wang, G., Khan, S., et al. (2013). Characterization and DNA-Binding Specificities of Ralstonia TAL-like Effectors. Mol Plant.
Loftus, S.K., Larson, D.M., Baxter, L.L., Antonellis, A., Chen, Y., Wu, X., Jiang, Y., Bittner, M., Hammer, J.A., 3rd, and Pavan, W.J. (2002). Mutation of melanosome protein RAB38 in chocolate mice. Proc. Natl. Acad. Sci. U.S.A. 99, 4471-4476.
Lopes, V.S., Wasmeier, C., Seabra, M.C., and Futter, C.E. (2007). Melanosome maturation defect in Rab38-deficient retinal pigment epithelium results in instability of immature melanosomes during transient melanogenesis. Mol. Biol. Cell 18, 3914-3927.
Maeder, M.L., Thibodeau-Beganny, S., Osiak, A., Wright, D.A., Anthony, R.M., Eichtinger, M., Jiang, T., Foley, J.E., Winfrey, R.J., Townsend, J.A., et al. (2008). Rapid "open-source" engineering of customized zinc-finger nucleases for highly efficient gene modification. Mol. Cell 31, 294-301.
Maeder, M.L., Thibodeau-Beganny, S., Sander, J.D., Voytas, D.F., and Joung, J.K. (2009). Oligomerized pool engineering (OPEN): an "open-source" protocol for making customized zinc-finger arrays. Nat Protoc 4, 1471-1501.
Meng, H., Hager, K., Rivkees, S.A., and Gruen, J.R. (2005). Detection of Turner syndrome using high-throughput quantitative genotyping. J. Clin. Endocrinol. Metab. 90, 3419-3422.
Meyer, M., de Angelis, M.H., Wurst, W., and Kuhn, R. (2010). Gene targeting by homologous recombination in mouse zygotes mediated by zinc-finger nucleases. Proc Natl Acad Sci U S A 107, 15022-15026.
Meyer, M., Ortiz, O., Hrabé de Angelis, M., Wurst, W., and Kühn, R. (2012). Modeling disease mutations by gene targeting in one-cell mouse embryos. Proc. Natl. Acad. Sci. U.S.A. 109, 9354-9359.
Miller, J.C., Tan, S., Qiao, G., Barlow, K.A., Wang, J., Xia, D.F., Meng, X., Paschon, D.E., Leung, E., Hinkley, S.J., et al. (2011). A TALE nuclease architecture for efficient genome editing. Nat. Biotechnol. 29, 143-148.
Nagy, A., Gertsenstein, M., Vintersten, K., and Behringer, R. (2003). Manipulating the Mouse Embryo (Cold Spring Harbour, New York: Cold Spring Harbour Laboratory Press).
Oiso, N., Riddle, S.R., Serikawa, T., Kuramoto, T., and Spritz, R.A. (2004). The rat Ruby ( R) locus is Rab38: identical mutations in Fawn-hooded and Tester-Moriyama rats derived from an ancestral Long Evans rat sub-strain. Mamm. Genome 15, 307-314.
Osanai, K., Higuchi, J., Oikawa, R., Kobayashi, M., Tsuchihara, K., Iguchi, M., Huang, J., Voelker, D.R., and Toga, H. (2010). Altered lung surfactant system in a Rab38-deficient rat model of Hermansky-Pudlak syndrome. Am. J. Physiol. Lung Cell Mol. Physiol. 298, L243-251.
Palais, R.A., Liew, M.A., and Wittwer, C.T. (2005). Quantitative heteroduplex analysis for single nucleotide polymorphism genotyping. Anal. Biochem. 346, 167-175.
Pâques, F., and Duchateau, P. (2007). Meganucleases and DNA double-strand break-induced recombination: perspectives for gene therapy. Curr Gene Ther 7, 49-66.
Pearson, W.R., and Lipman, D.J. (1988). Improved tools for biological sequence comparison. Proc. Natl. Acad. Sci. U.S.A. 85, 2444-2448.
Peippo, J., Viitala, S., Virta, J., Räty, M., Tammiranta, N., Lamminen, T., Aro, J., Myllymäki, H., and Vilkki, J. (2007). Birth of correctly genotyped calves after multiplex marker detection from bovine embryo microblade biopsies. Mol. Reprod. Dev. 74, 1373-1378.
Petersen, D.C., Laten, A., Zeier, M.D., Grimwood, A., Rensburg, E.J. van, and Hayes, V.M. (2002). Novel mutations and SNPs identified in CCR2 using a new comprehensive denaturing gradient gel electrophoresis assay. Hum. Mutat. 20, 253-259.
Di Pietro, S.M., and Dell'Angelica, E.C. (2005). The cell biology of Hermansky-Pudlak syndrome: recent advances. Traffic 6, 525-533.
Porteus, M.H., and Baltimore, D. (2003). Chimeric nucleases stimulate gene targeting in human cells. Science 300, 763.
Porteus, M.H., and Carroll, D. (2005). Gene targeting using zinc finger nucleases. Nat Biotechnol 23, 967-973.
Ramon, D., Braden, M., Adams, S., Marincola, F.M., and Wang, L. (2003). Pyrosequencing™: A one-step method for high resolution HLA typing. J Transl Med 1, 9.
Remigi, P., Anisimova, M., Guidot, A., Genin, S., and Peeters, N. (2011). Functional diversification of the GALA type III effector family contributes to Ralstonia solanacearum adaptation on different plant hosts. New Phytol. 192, 976-987.
Reyon, D., Tsai, S.Q., Khayter, C., Foden, J.A., Sander, J.D., and Joung, J.K. (2012). FLASH assembly of TALENs for high-throughput genome editing. Nat. Biotechnol. 30, 460-465.
Rouet, P., Smih, F., and Jasin, M. (1994a). Expression of a site-specific endonuclease stimulates homologous recombination in mammalian cells. Proc Natl Acad Sci U S A 91, 6064-6068.
Rouet, P., Smih, F., and Jasin, M. (1994b). Introduction of double-strand breaks into the genome of mouse cells by expression of a rare-cutting endonuclease. Mol Cell Biol 14, 8096-8106.
Salanoubat, M., Genin, S., Artiguenave, F., Gouzy, J., Mangenot, S., Arlat, M., Billault, A., Brottier, P., Camus, J.C., Cattolico, L., et al. (2002). Genome sequence of the plant pathogen Ralstonia solanacearum. Nature 415, 497-502.
Santiago, Y., Chan, E., Liu, P.Q., Orlando, S., Zhang, L., Urnov, F.D., Holmes, M.C., Guschin, D., Waite, A., Miller, J.C., et al. (2008). Targeted gene knockout in mammalian cells by using engineered zinc-finger nucleases. Proc Natl Acad Sci U S A 105, 5809-5814.
Schwartzberg, P.L., Goff, S.P., and Robertson, E.J. (1989). Germ-line transmission of a c-abl mutation produced by targeted gene disruption in ES cells. Science 246, 799-803.
Smith, T.F., and Waterman, M.S. (1981). Identification of common molecular subsequences. J. Mol. Biol. 147, 195-197.
Sung, Y.H., Baek, I.-J., Kim, D.H., Jeon, J., Lee, J., Lee, K., Jeong, D., Kim, J.-S., and Lee, H.-W. (2013). Knockout mice created by TALEN-mediated gene targeting. Nat. Biotechnol. 31, 23-24.
Tesson, L., Usal, C., Menoret, S., Leung, E., Niles, B.J., Remy, S., Santiago, Y., Vincent, A.I., Meng, X., Zhang, L., et al. (2011). Knockout rats generated by embryo microinjection of TALENs. Nat Biotechnol 29, 695-696.
Thomas, K.R., and Capecchi, M.R. (1987). Site-directed mutagenesis by gene targeting in mouse embryo-derived stem cells. Cell 51, 503-512.
Todd, R., Donoff, R.B., Kim, Y., and Wong, D.T. (2001). From the chromosome to DNA: Restriction fragment length polymorphism analysis and its clinical application. J. Oral Maxillofac. Surg. 59, 660-667.
Tost, J., and Gut, I.G. (2005). Genotyping single nucleotide polymorphisms by MALDI mass spectrometry in clinical applications. Clin. Biochem. 38, 335-350.
Wasmeier, C., Romao, M., Plowright, L., Bennett, D.C., Raposo, G., and Seabra, M.C. (2006). Rab38 and Rab32 control post-Golgi trafficking of melanogenic enzymes. J. Cell Biol. 175, 271-281.
Youil, R., Kemper, B.W., and Cotton, R.G. (1995). Screening for mutations by enzyme mismatch cleavage with T4 endonuclease VII. Proc. Natl. Acad. Sci. U.S.A. 92, 87-91.

## Claims

1. A method of producing a eukaryotic cell carrying a modified target sequence in its genome, the method comprising the step:
(a) introducing into the cell a fusion protein comprising a DNA-binding domain of a *Ralstonia solanacearum* TALE-like protein (RTL) effector protein and a non-specific cleavage domain of a restriction nuclease or a nucleic acid molecule encoding the fusion protein in expressible form, wherein the fusion protein specifically binds within the target sequence and introduces a double strand break within the target sequence.

2. The method of claim 1, wherein the modification of the target sequence is by homologous recombination with a donor nucleic acid sequence further comprising the step:
(b) introducing a nucleic acid molecule into the cell, wherein the nucleic acid molecule comprises the donor nucleic acid sequence and regions homologous to the target sequence.

3. The method of claim 1 or 2, wherein the cell is selected from the group consisting of a mammalian or vertebrate cell, or a fungal cell.

4. The method of any one of claims 1 to 3, wherein the cell is an oocyte.

5. The method of any one of claims 1 to 4, wherein the fusion protein or the nucleic acid molecule encoding the fusion protein is introduced into the cell by microinjection.

6. The method of any one of claims 2 to 4, wherein the nucleic acid molecule of (b) is introduced into the cell by microinjection.

7. The method of any one of claims 1 to 6, wherein the nucleic acid molecule encoding the fusion protein in expressible form is mRNA.

8. The method of any one of claims 2 to 7, wherein the regions homologous to the target sequence are localised at the 5' and 3' end of the donor nucleic acid sequence.

9. The method of any one of claims 2 to 8, wherein the regions homologous to the target sequence comprised in the nucleic acid molecule of (b) have a length of at least 400 bp.

10. The method of any one of claims 1 to 9, wherein the modification of the target sequence is selected from the group consisting of substitution, insertion and deletion of a least one nucleotide of the target sequence.

11. The method of any one of claims 1 to 10, wherein the cell is from a mammal selected from the group consisting of rodents, dogs, felides, primates, rabbits, pigs, and ruminants or wherein the cell is from an avian selected from the group consisting of chickens, turkeys, pheasants, ducks, geese, quails and ratites including ostriches, emus and cassowaries or wherein the cell is from zebrafish.

12. A method of producing a non-human vertebrate or mammal carrying a modified target sequence in its genome, the method comprising the steps of
(a) producing a cell in accordance with any one of claims 1 to 11;
(b) transferring the cell obtained in (a) into a pseudo pregnant female host; and
(b) analysing the offspring delivered by the female host for the presence of the modification.

13. The method of claim 12, further comprising culturing the cell to form a pre-implantation embryo or introducing the cell into a blastocyst prior to transferring it into the pseudopregnant female host.

14. The method of claim 12 or 13, wherein the non-human mammal is selected from the group consisting of rodents, dogs, felides, primates, rabbits, pigs and ruminants or wherein the vertebrate is selected from the group consisting of fish and avians.

15. A fusion protein comprising a DNA-binding domain of a RTL effector protein and a non-specific cleavage domain of a restriction nuclease.
